(19) Europäisches Patentamt
European Patent Office
Office européen des brevets

(11) Publication number: **0 087 564**
**A1**

(12) # EUROPEAN PATENT APPLICATION

(21) Application number: 83100413.0

(22) Date of filing: 19.01.83

(51) Int. Cl.$^3$: G 01 N 33/58

(30) Priority: 01.02.82 US 344607

(43) Date of publication of application:
07.09.83 Bulletin 83/36

(84) Designated Contracting States:
DE FR GB IT

(71) Applicant: MILES LABORATORIES INC.
1127 Myrtle Street
Elkhart Indiana 46514(US)

(72) Inventor: Buckler, Robert Thomas
23348 May Street
Edwardsburg, MI 49112(US)

(72) Inventor: Li, Thomas M.
2914 Brooktree Court
Elkhart, IN 46514(US)

(74) Representative: Senftl, Hannes, Dr. et al,
c/o Bayer AG Zentralbereich Patente Marken und Lizenzen
D-5090 Leverkusen 1, Bayerwerk(DE)

(54) **Specific binding assay method, reagent system and labelled conjugate for use in this method.**

(57) An improved specific binding assay, e.g., homogeneous or heterogeneous immunoassay, for determining an analyte employing a labeled conjugate of said analyte and a photogenic, e.g., fluorescent, label. Where the analyte in the analyte-photophor conjugate whose light emission is measured in the assay as a function of the analyte is capable of significantly reducing such light emission upon coming into quenching orientation with the photophor, the present invention employs a labeled conjugate in which the analyte is joined to the photogenic label by a linking group which substantially hinders such quenching orientation. In this manner, ten percent or more of the photogenicity of the unconjugated photophor can be preserved in the measured analyte-photophor conjugate. The use of conventional flexible linking groups in the labeled conjugate allows quenching to occur with attendant large losses in assay sensitivity, whereas sensitivity is increased in the present invention by the use of relatively rigid linking groups. The invention is applicable to a wide variety of photogenic, e.g., fluorescent and chemiluminescent, binding assay techniques.

EP 0 087 564 A1

Croydon Printing Company Ltd.

# SPECIFIC BINDING ASSAY METHOD, REAGENT SYSTEM AND LABELED CONJUGATE FOR USE IN THIS METHOD

## BACKGROUND OF THE INVENTION

### *1. FIELD OF THE INVENTION*

The development of specific binding assay techniques has provided extremely useful analytical methods for determining various organic substances of diagnostic, medical, environmental and industrial importance which appear in liquid mediums at very low concentrations. Specific binding assays are based on the specific interaction between the substance under determination, herein referred to as analyte, and a binding partner thereof. Where one of the analyte and its binding partner is an antibody and the other is a corresponding hapten or antigen, the assay is known as an immunoassay. Other binding interactions between the analyte and a binding partner serve as the bases of binding assays, including the binding interactions between hormones, vitamins, metabolites, and pharmacological agents, and their respective receptors and binding substances.

In conventional specific binding assay techniques, a test sample to be assayed is combined in a liquid reaction mixture with reagent systems of various compositions. Such systems usually comprise (i) a labeled conjugate, which is a conjugate of the analyte, or a specific binding analog thereof, and a labeling substance, and (ii) a limiting amount of a binding partner for the analyte. In the reaction mixture then, analyte in the sample and labeled conjugate compete for binding to the binding partner resulting in formation of a binding partner-bound-species and a free-species of the labeled conjugate. The relative amount or proportion of the labeled conjugate that results in the bound-species compared to the free-species is a function of the presence (or amount) of the analyte in the test sample. One can thus measure the labeling substance in the free-species and correlate the measured amount with the presence or amount of the analyte in the sample.

Where the labeled conjugate in the bound-species is essentially indistinguishable in the presence of the labeled conjugate in the free-species by the means used to monitor the label, the bound-species and the free-species must be physically separated in order to complete the assay. This type of assay is referred to in the art as "heterogeneous". Where the bound-species and free-species forms of the labeled conjugate can be distinguished in the presence of each other, a "homogeneous" format can be followed and the separation step avoided.

The present invention relates to homogeneous and heterogeneous specific binding assay methods and reagent systems for the quantitative or qualitative determination of an analyte in a test sample involving the use of photogenic labels, e.g., chemiluminescers and

fluorescers. In particular, this invention relates to such methods and systems of the homogeneous type, particularly those employing fluorogenic enzyme substrate-active labels. The present invention additionally relates to photogen- and photophor-labeled conjugates used in such assay methods and reagent systems.

## 2. *DESCRIPTION OF THE PRIOR ART*

The first highly sensitive specific binding assay to be discovered was the radioimmunoassay which employs a radioactive isotope as the label. Such an assay necessarily must follow the heterogeneous format since the monitorable character of the label is qualitatively unchanged in the free- and bound-species. Because of the inconvenience and difficulty of handling radioactive materials, assay systems have been devised using materials other than radioisotopes as the label component, including cofactors, enzyme substrates, enzyme modulators, e.g., activators and inhibitors, cycling reactants, spin radicals, enzymes, bacteriophages, metals and organometallic complexes, organic and inorganic catalysts, prosthetic groups, chemiluminescent reactants, and fluorescent molecules.

Among such specific binding assay techniques are those which employ photogenic labels, most noteably, chemiluminescers and fluorescers. In such assays, the free-species of the labeled conjugate yields an analyte-photophor conjugate comprising the analyte or its analog linked to a photophor capable of emitting light upon excitation by appropriate means. The sensitivity of such an assay is dependent on the ability to photogenically measure the photophor label without interference from the analyte or analog portion of the

conjugate. If the analyte or its analog in the conjugate is of a type which can significantly diminish the photogenicity of the photophor label when proximate to the photophor, the sensitivity of any assay for such an analyte would suffer greatly.

It is an object of the present invention to provide a means for alleviating such analyte-induced quenching of photogenic labels employed in certain specific binding assays, particularly those of the homogeneous type.

Homogeneous photogenic enzyme-substrate-labeled specific binding assays are described by Burd *et al*, *Anal. Biochem.* *77*:56(1977) and in German OLS 2,618,511 and British Pat. No. 1,552,607 corresponding to U.S. Serial No. 667,996, filed March 18, 1976, and assigned to the present assignee. An improved technique is described by Burd *et al*, *Clin. Chem.* *23*:1402(1977) and in U.S. Pat. No. 4,279,992. Reference is made in Csiszar *et al*, *Clin. Chem.* *27*:1087 (1981) to the application of such technique to an assay for quinidine.

Other homogeneous specific binding assays employing photogenic labels that are reported in the literature include those based on the following phenomena: fluorescence polarization [McGregor *et al*, *Clin. Chim. Acta* *83*:161(1978)], fluorescence quenching [Shaw *et al*, *J. Clin. Pathol.* *30*:526(1977)], fluorescence enhancement [Smith, *FEBS Letters* *77*:25(1977)], energy transfer [Ullman *et al*, *J. Biol. Chem.* *251*:4172(1978) and U.S. Pat. No. 3,996,345], chemically-excited fluorescence [U.S. Pat. No. 4,238,195], and fluorescence protection [Zuk *et al*, *Clin. Chem.* *25*:1554(1979) and U.S. Pat. Nos. 3,935,074 and 3,998,943].

Heterogeneous specific binding assays employing photogenic labels are described in U.S. 3,720,760; 3,940,475; 3,992,631; 4,058,732; 4,133,639; 4,144,452;

4,171,311 and 4,201,763. A review of fluorescence immunoassay techniques is provided by Visor *et al*, *J. Pharm. Sci. 70*:469(1981).

Of ancillary relevance to the present invention are Weber, *Flavins and Flavoproteins*, ed. Slater, Elsevier (Amsterdam 1966) pp. 15-21 and Forster, *Ann. Phys. 2*:55(1948) relating to the phenomena of intramolecular collision and energy transfer; Lakowicz *et al*, *Biochem. 12*:4161(1973) relating to the phenomenon of static quenching; Spencer *et al*, *Structure and Function of Oxidation Reduction Enzymes*, ed. Akeson et al, Pergamon Press (New York 1972) pp. 393-399 relating to the use of fluorescence yields and lifetimes in assigning the relative proportions of open and stacked conformations in the intramolecular complex of flavin adenine dinucleotide; European Pat. Appl. 15,695 and Ullman *et al*, "Homogeneous Fluorescence Immunoassays" in *Immunoassays:Clinical Laboratory Techniques for the 1980s*, ed. Nakamura et al, Alan R. Liss (New York 1980) pp. 13-43 relating to the conjugation of analyte-fluorescer to rigid macromolecular supports to reduce fluorescence interference due to nonspecific protein binding in certain immunoassays.

## SUMMARY OF THE INVENTION

The present invention provides an improvement in specific binding assays employing photogenic labels for the determination of analytes which have the capacity to quench light emission from analyte-photophor conjugates either used in or generated during the assay. The improvement is accomplished by employing a labeled conjugate in which the analyte or its analog is joined to the photogenic label by a linking group which substantially hinders (i.e., disallows to a significant degree) quenching orientation of the photophor with

the analyte or analog portion in the analyte-photophor conjugate whose light emission is measured in the assay. The invention is applicable to any type of homogeneous or heterogeneous specific binding assay, most particularly homogeneous immunoassays, wherein the assay is monitored, i.e., a detectable signal is correlated with analyte in the sample tested, by measuring light emission from a photophor, particularly a fluorescer, conjugated chemically to a quenching analyte or analog thereof. The analyte-photophor conjugate whose light emission is measured may be identical with the photogenic labeled conjugate combined as an assay reagent with the test sample or may be a product of a chemical reaction involving such photogenic labeled conjugate reagent, e.g., the product of enzyme modification of the photogenic labeled conjugate.

The sensitivity of photogenic specific binding assays is markedly improved when the linking group hinders the quenching mode between the photophor and analyte portions of the conjugate sufficient to observe 10%, more preferably 15%, and most preferably 20%, of the photogenicity of the unconjugated photophor in the conjugate. Alleviation of analyte-induced quenching is accomplished by including in the linking group a portion of sufficient rigidity to substantially restrict the rotational degrees of freedom between the analyte and the photophor.

## BRIEF DESCRIPTION OF THE DRAWINGS

Fig. 1 shows the chemical structures of particular fluorogenic quinidine conjugates prepared and used in Example 1.

Figs. 2 and 2a show the chemical structures of particular fluorogenic iodothyronine, e.g., thyroxine, conjugates prepared and used in Example 2.

## DESCRIPTION OF THE PREFERRED EMBODIMENTS

In the context of this disclosure, the following terms shall be defined as follows unless otherwise indicated:

Analyte - the substance, or class of related substances, whose presence or amount in a test sample is under determination.

Binding counterpart of the analyte - any substance, or class of substances, which has a specific binding affinity, normally reversible, for the analyte.

Specific binding analog of the analyte - any substance, or class of substances, which behave similarly to the analyte with respect to binding by a binding counterpart of the analyte.

Reagent system - a composition, test device, test kit, or other physical arrangement, means, or combination of reagents for use in performing the present assay.

### *ANALYTE*

The present assay may be applied to any analyte detectable by photogenic specific binding assay methods and which exhibits undesirable quenching of the photophor in the measured analyte-photophor-conjugate. The propensity of an analyte to quench a particular photophor can be assessed by Stern-Volmer studies [Stern and Volmer, *Z. Phys.* *20*:183(1919) and Vaughan and Weber, *Biochem.* *9*:464(1970)]. In such studies, increasing concentrations of a potential quencher are added to a constant level of the unconjugated photophor and the effect on fluorescence is monitored.

The analyte usually is a peptide, polypeptide, protein, carbohydrate, glycoprotein, steroid, or other organic molecule for which a specific binding counterpart exists in biological systems or can be synthesized. The analyte, in functional terms, is usually selected from the group comprising antigens and antibodies thereto; haptens and antibodies thereto; and hormones, vitamins, metabolites and pharmacological agents, and their binding counterparts. Usually, the analyte is an immunologically-active polypeptide or protein, usually having a molecular weight of between about 1,000 and about 10,000,000, such as an antibody or antigenic polypeptide or protein, or a hapten having a molecular weight of at least about 100, and usually less than about 1,500.

Representative polypeptide analytes are angiotensin I and II, C-peptide, oxytocin, vasopressin, neurophysin, gastrin, secretin, bradykinin, and glucagon.

Representative protein analytes include the classes of protamines, mucoproteins, glycoproteins, globulins, albumins, scleroproteins, phosphoproteins, histones, lipoproteins, chromoproteins, and nucleoproteins. Examples of specific proteins are prealbumin, $\alpha_1$-lipoprotein, human serum albumin, $\alpha_1$-acid glycoprotein, $\alpha_1$-antitrypsin, $\alpha_1$-glycoprotein, transcortin, thyroxine binding globulin, haptoglobin, hemoglobin, myoglobin, ceruloplasmin, $\alpha_2$-lipoprotein, $\alpha_2$-macroglobulin, β-lipoprotein, erythropoietin, transferin, hemopexin, fibrinogen, the immunoglobulins such as IgG, IgM, IgA, IgD, and IgE, and their fragments, e.g., $F_c$ and $F_{ab}$, complement factors, prolactin, blood clotting factors such as fibrinogen, thrombin and so forth, insulin, melanotropin, somatotropin, thyrotropin, follicle stimulating hormone,

leutinizing hormone, gonadotropin, thyroid stimulating hormone, placental lactogen, intrinsic factor, transcobalamin, serum enzymes such as alkaline phosphatase, lactic dehydrogenase, amylase, lipase, phosphatases, cholinesterase, glutamic oxaloacetic transaminase, glutamic pyruvic transaminase, and uropepsin, endorphins, enkephalins, protamine, tissue antigens, bacterial antigens, and viral antigens such as hepatitis associated antigens (e.g., $HB_sAg$, $HB_cAg$ and $HB_eAg$).

The present invention is particularly useful in the assay of hapten analytes, e.g., having molecular weights less than about 1500. Representative hapten analytes include the general classes of drugs, metabolites, hormones, vitamins, and the like organic compounds. Haptenic hormones include the iodothyronines such as thyroxine and triiodothyronine. Vitamins include vitamins A, B, e.g., $B_{12}$, C, D, E and K, folic acid and thiamine. Drugs include antibiotics such as aminoglycosides, e.g., gentamicin, tobramycin, amikacin, sisomicin, kanamycin, and netilmicin, penicillin, tetracycline, terramycin, chloromycetin, and actinomycetin; nucleosides and nucleotides such as adenosine diphosphate (ADP) adenosine triphosphate (ATP), flavin mononucleotide (FMN), flavin adenine dinucleotide (FAD), nicotinamide adenine dinucleotide (NAD) and its phosphate derivative (NADP), thymidine, guanosine and adenosine; prostaglandins; steroids such as the estrogens, e.g., estriol and estradiol, sterogens, androgens, digoxin, digitoxin, and adrenocortical steroids; and others such as phenobarbital, phenytoin, primidone, ethosuximide, carbamazepine, valproate, theophylline, caffeine, propranolol, quinidine, amitriptyline, cortisol, desipramine, disopyramide,

doxepin, doxorubicin, nortriptyline, methotrexate, imipramine, lidocaine, procainamide, N-acetylprocainamide, amphetamines, catecholamines, and antihistamines.

## *QUENCHING EFFECT*

The present invention is predicated on the use of linking groups that hinder, i.e., prevent or inhibit to an analytically significant degree, quenching of the photogenicity, i.e., the ability to emit light upon excitation, of the photophor in the measured analyte-photophor conjugate by the analyte or analog portion of the conjugate. The analyte-induced quenching effect can be based on any physical, chemical or electrical interaction between the photophor and the analyte portion and is usually based on intramolecular modulation, particularly that due essentially to either direct intramolecular collision or resonance energy transfer. Interaction between the photophor and the analyte portion results in photophor quenching when the analyte portion assumes a proximate quenching orientation with the photophor.

### *1. Direct Intramolecular Collision*

In this quenching phenomenon, modulation of the photogenicity of the photophor can result from static or dynamic processes. Static quenching results when the photophor and quencher interact prior to excitation of the photophor, whereas dynamic quenching results from interaction with the excited photophor whereby the quenching mechanism competes with emission for depopulation of the excited state.

Dynamic and static quenching require short range collision or contact interaction between the photophor and quencher.

The principles of direct intramolecular collision quenching are described in the literature [Vaughan *et al*, *Biochem.* *9*:464(1970)] and have been applied to analytical chemistry [Guilbault, *Fluorescence, Instrumentation and Practice*, Decker (New York 1967) p. 349], the study of membrane proteins [Shinitzky *et al*, *Biochem.* *16*:982(1977)], the investigation of liposome-cell interactions [Weinstein *et al*, *Science* *195*:489(1977)], and enzyme assays [Yaron *et al*, *Anal. Biochem.* *95*:228(1979)]. Incorporation of a quenching analyte or analog into the labeled conjugate such that a high frequency of collisions with the photophor is allowed to occur provides a system exhibiting direct intramolecular collision quenching. Selection of a linking group which restrains movement of the opposed photophor and analyte portions of the conjugate reduces the frequency of collisions and thereby relieves photophor quenching by this mechanism.

Quenching by direct intramolecular collision is particularly prone to occur between the photophor and analyte portions of the conjugate where both parties are aromatic in character. Strong ring to ring interactions become likely to occur, leading to molecular stacking configurations which promote quenching. Additionally, when the analyte contains heavy atoms, the enhanced rate of intersystem crossing to the triplet state which competes with fluorescence decay can lead to quenching.

*2. Resonance energy transfer*

In this mechanism, energy is transferred from the photophor to a quencher over an intramolecularly long distance, as long as 60Å, but preferably less than 25Å. The theory of resonance energy transfer has been established by Forster, *Ann. Phys.* 2:55(1948). Unlike direct intramolecular quenching, efficiency of quenching due to resonance energy transfer is dependent upon the spectral overlap of the emission spectrum of the photophor with the absorption spectrum of the quencher. This quenching phenomenon has been applied to enzyme assays [Yaron *et al, Anal. Biochem. 95*:228(1970)] and immunoassays [Ullman *et al, J. Biol. Chem. 251*:4172 (1976) and U.S. Patent No. 3,996,345]. Selection of a linking group which restricts the ability of the opposed photophor and analyte portions of the conjugate to come sufficiently close for resonance energy transfer to occur results in relief of photophor quenching by this mechanism.

Accordingly, the phrase "quenching orientation" is used herein to be generic to the quenching mechanisms of direct intramolecular collision and resonance energy transfer.

*PHOTOGENIC LABELED CONJUGATE*

The labeled conjugate refers to the reagent entity combined with the test sample and the other assay reagents, which conjugate comprises the analyte, or a specific binding analog thereof, and a photogenic label. By photogenic label is intended a material which itself is a photophor, i.e., is itself capable of light emission upon excitation, or is a precursor

of a photophor, i.e., is a residue which upon chemical reaction is modified to produce a photophor. Thus, the photogenic labeled conjugate reagent may be identical with the photophor-analyte conjugate whose photogenicity is measured to complete the assay or may be a precursor thereto. If it is a precursor, the labeled conjugate will undergo chemical reaction, usually enzyme-catalyzed, which modifies the photogenic-label portion to render it a photophor. For example, a nonfluorescent fluorescer derivative coupled to an analyte or analog would serve as a fluorogenic labeled conjugate if upon chemical reaction a fluorescent fluorescer-analyte conjugate is generated.

The photophor in the measured analyte-photophor conjugate can be any molecular entity which can be stimulated to emit light by exposure to excitation means of any physical, chemical, or electrical nature, e.g., irradiation with light or exposure to chemical reaction. The principal categories of the photophor are fluorescers, phosphorescers, and chemiluminescers. Fluorescers and phosphorescers are molecules which upon irradiation with light of a predetermined first wavelength emit light of a second, longer wavelength. Chemiluminescers emit light upon chemical reaction which may or may not be enzyme catalyzed.

Examples of useful fluorescers are umbelliferone (7-hydroxycoumarin), fluorescein, indole, β-naphthol, 3-pyridol, resorufin, lissamine rhodamine B, rhodamine B, anthracene, 3,10-diphenylanthracene, perylene, rubrene, pyrene, tryptophan, flavin, acridine, naphthalene, α-naphthylamine, 5-dimethylamino-1-naphthalenesulfonate (dansyl), N-[*p*-(2-benzoxazoyl)phenyl]maleimide, thiochrome, anilinosulfonic acid, and their various fluorescent derivatives. Examples

of phosphorescers are quinoline, 1-bromonaphthalene, indole, tryptophan, tyrosine, and their various phosphorescent derivatives. See Meyers *et al*, *Anal. Chem.* *51*:1609(1979). Examples of useful chemiluminescers are luminol, isoluminol, luciferin, 2,3-dihydrobenzo[g] phthalazine-1,4-dione, 2,3-dihydrophthalazine-1,4-dione, and their various chemiluminescent derivatives.

*LINKING GROUPS*

As in conventional labeled conjugates, the photogenic label and the analyte, or its analog, will be joined covalently by a chain comprising from 1 to 50 atoms, more commonly 1 to 30 atoms, and usually 1 to 20 atoms, excluding hydrogen, principally carbon and heteroatoms selected from nitrogen, oxygen, phosphorous, and sulfur. Conventional linking groups are described at length in the literature. See, for example, U.S. Pat. Nos. 4,230,797; 4,279,992; 3,817,837; 3,935,074; and 3,996,345. The present invention departs from convention in selecting a relatively rigid or only partially flexible residue for insertion into such conventional linking groups in order to impart sufficient rigidity to significantly reduce quenching interaction between the photophor and analyte portions. Once aware of the solution afforded by the present invention to the potential quenching problems of the prior art, one skilled in the art will have a variety of chemical groupings and residues from which to choose to impart the desired rigidity to the conventional linking groups. A sampling of such groupings and residues are provided below:

| Linking arm type | Structural examples |
|---|---|
| piperazinylene, particularly the depicted N,N'-substituted form | 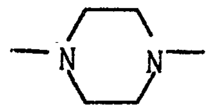 |
| phenylene, particularly the depicted *para*-substituted form | 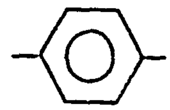 |
| cyclohexylene, particularly the depicted 1,4-substituted form | 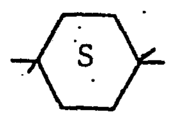 |
| carbocyclic and heterocyclic fused-ring systems, e.g., naphthalenes, anthracenes, *et al* | 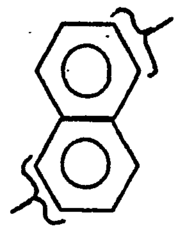 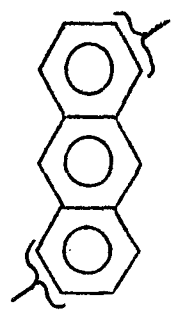 |

| Linking arm type | Structural examples |
| --- | --- |
| acetylene | $-C \equiv C-$ |
| *bis*-substituted monosaccharides | 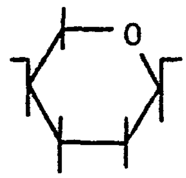 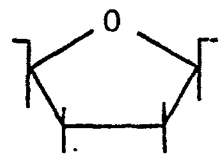 |
| bicyclo[2.2.2]octane rod, *J. Org. Chem. 45*: 3934(1980) | 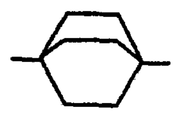 |
| spiro-linked ring structures *JACS 90*:1940 (1968) | 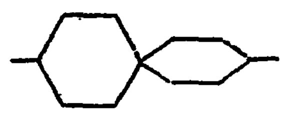 |

Other systems are described in *Proc. Natl. Acad. Sci. USA 58*:719(1967) [poly-L-proline], *JACS 91*:1293(1969) [saturated steroids], *JACS 87*:995(1965) [decacyclic bisteroid system], *JACS 90*:6897(1968) [saturated fused-ring network], *Helv. Chem. Acta 58*:397(1975) [bicyclic hydrocarbon frame], *JACS 90*:6425(1968), *JACS 91*:4786(1969); and *JACS 91*:4169(1969)

On either side of the rigid portion within the linking group will usually be conventional linking structures coupling to the photophor and analyte portions respectively.

## *ASSAY TECHNIQUES*

In broad principle, the present invention is applicable to any homogeneous or heterogeneous specific binding assay technique which employs a labeled conjugate comprising a photogenic label and the analyte or an analog thereof. In any such technique, if the analyte is of a type which can quench the photogenic label, the total potential signal from the analyte-photophor conjugate measured in the assay will be correspondingly reduced, thereby decreasing the sensitivity of the assay, i.e., the lower limits of analyte concentrations that can be reproducibly detected.

### *A. Homogeneous Formats*

Generally speaking, the present assay method involves the steps of (1) combining the test sample in a liquid reaction mixture with a reagent system including a binding partner for the analyte and the labeled conjugate as described herein, resulting in the formation in the reaction mixture of a binding partner-bound species and a free-species (i.e., not bound by binding partner) of the labeled conjugate, the free-species yielding the analyte-photophor conjugate (either as the labeled conjugate itself labeled conjugate, the free-species yielding the analyte-photophor conjugate (either as the labeled conjugate itself or as a reaction product from the labeled conjugate), and (2) exciting the photophor by appropriate means and measuring the resulting light emitted as a function of the analyte in the test sample. Following are examples, without limitation, of particular homogeneous assay techniques to which the present invention applies:

*1. Enzyme substrate-labeled technique*

In this system, the labeled conjugate comprises the analyte or analog and a photogenic, e.g., fluorogenic, enzyme substrate-active label, wherein such label is capable of being acted on by an enzyme to produce the analyte-photophor conjugate. The ability of the enzyme to act on the labeled conjugate to release the detected analyte-photophor conjugate is decreased by binding of the labeled conjugate with the binding partner recognizing the analyte (commonly an antibody or a fragment thereof). Assay systems of this type are included in the principle described in commonly assigned, copending application Serial No. 894,836, filed April 10, 1978 (corresponding to U.K. Pat. Spec. 1,552,607).

In such enzyme substrate-labeled techniques, the labeled conjugate, e.g., a substrate-analyte conjugate, will have the property that it can be acted upon by an enzyme, by cleavage or modification, to produce a product having a detectable property which distinguishes it from the conjugate. For example, the conjugate may be nonfluorescent under assay conditions but upon reaction with enzyme a fluorescent analyte-fluorescer product is produced.

Various fluorogenic substrate-labeled conjugates are evident for use in such techniques. For example, the labeled conjugate may be of the formula:

G-D-R-L

wherein G is a cleavable group such as phosphate, carboxylate, or glycone, D is a fluorogenic dye moiety which upon removal of G yields a fluorescent product, e.g., D can be umbelliferone, fluorescein, rhodamine, and their derivatives, R is the linking group and L is the analyte or analog thereof. Enzymatic cleavage (e.g., by phosphatase, carboxylase, glycosidase, etc.) of the labeled conjugate is affected by binding, such as by antibody, to the analyte L portion of the conjugate. See U.S. Pat. No. 4,279,992. A particularly preferred substrate-labeled assay scheme employs a labeled conjugate of the type:

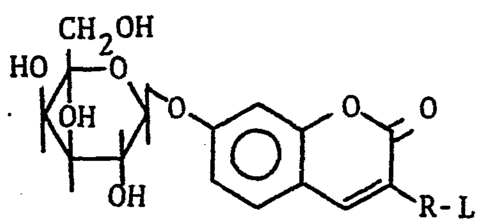

wherein R and L are as above, whereby the ability of the enzyme β-galactosidase to cleave the conjugate yielding a product distinguishable by its fluorescence is inhibited by binding of the conjugate with antibody.

An example of a rigid portion inserted into linking group R for the purposes of the present invention is *para*-phenylene, yielding conjugates of the general formula:

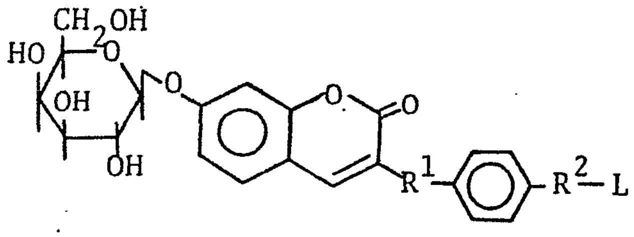

wherein $R^1$ and $R^2$ are appropriate bridging elements. Conjugates of this general type are represented by the specific structure (*4*)

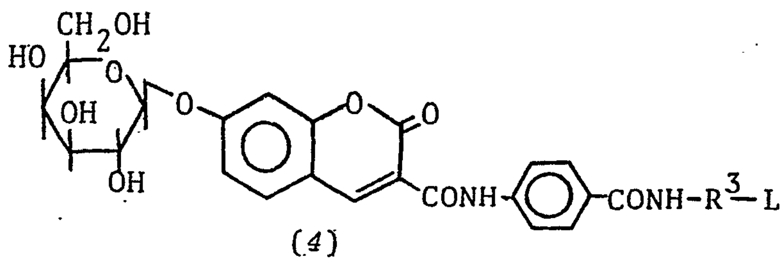

(*4*)

where $R^3$ is an appropriate bridging element. e.g., a bond or a chain of from 1-20 atoms excluding hydrogen, and L is as above. Conjugates of this type can be prepared as outlined in Scheme A. Here, 7-β-galactosylcoumarin-e-carboxylic acid (*1*) [Burd *et al*, *Clin. Chem* *23*:1402(1977)] can be converted to the activated ester (*2*) by reaction with N-hydroxysuccinimide and dicyclohexylcarbodiimide in DMF at room temperature for 2 hours [Anderson *et al*, *J. Amer. Chem. Soc.* *86*:1839(1964)]. The activated ester (*2*) is then combined in a suitable solvent, such as DMF, with *para*-aminobenzoic acid in the presence of triethylamine and stirred for 24 hours at room temperature. The reaction mixture is then filtered to remove the byproduct dicyclohexyl urea and the

Scheme A

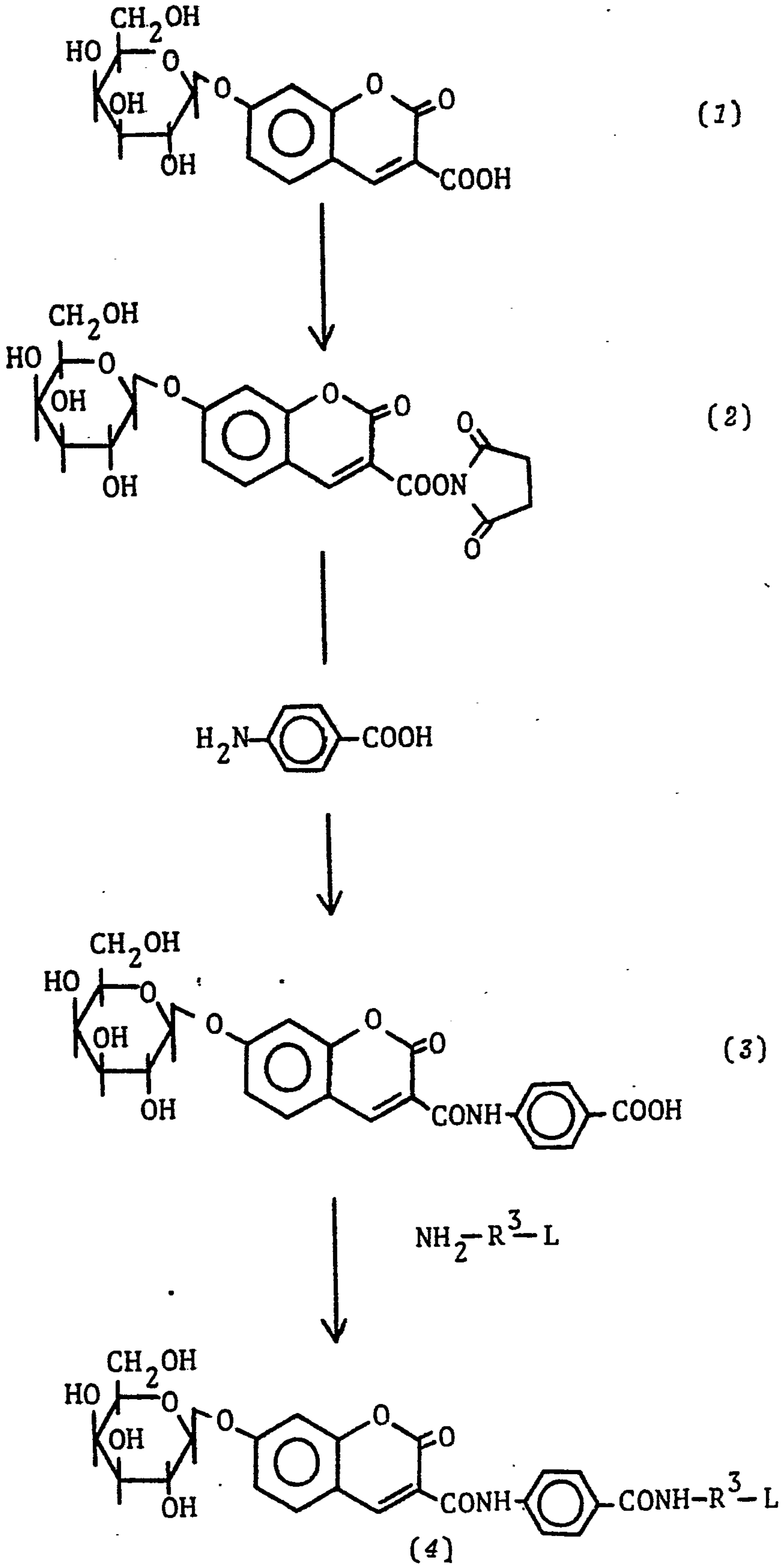


(1)

(2)

(3)

(4)

solvent evaporated under high vacuum to give a residue from which pure N-(*para*-carboxyphenyl)-7-β-galactosylcoumarin-3-carboxamide, (*3*), can be obtained, for example, by chromatography on silicic acid and eluting with ethanol-ethyl acetate mixtures. Label (*3*) can be attached to an amino-modified or amino-containing ligand by appropriate amide and peptide bond forming reactions [c.f. "Peptides" Goodman and Meienhofer, ed., John Wiley & Sons (New York 1977), pp. 6-13 and references cited therein].

Another example of a rigid portion inserted into linking group R for the purpose of the present invention is 1,4-disubstituted cyclohexyl, yielding conjugates of the general formula:

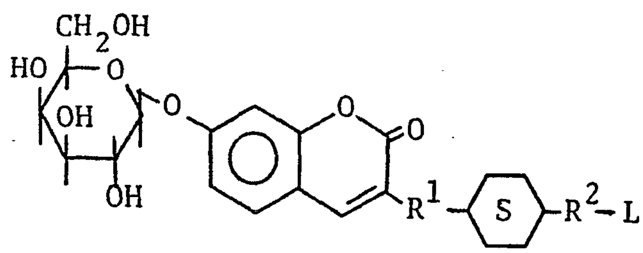

wherein $R^1$ and $R^2$ are appropriate bridging elements. Conjugates of this general type are represented by the specific structure (*5*)

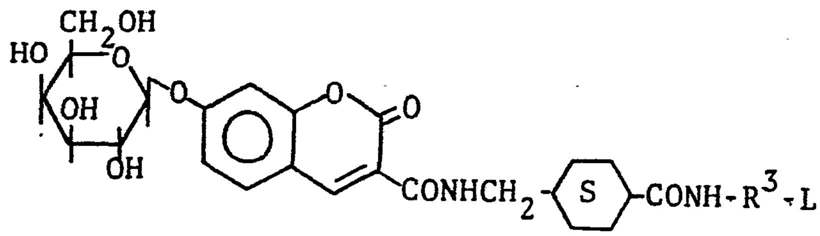

(*5*)

where $R^3$ is an appropriate bridging element, e.g., a bond or a chain of from 1-20 atoms excluding hydrogen, and L is as above. Conjugates of this type can be prepared by reacting the activated ester (*2*) with *trans*-4-(aminomethyl)cyclohexane carboxylic acid, under the conditions described for the synthesis of (*3*), to give the acid N-(*trans*-4-carboxycyclohexylmethyl)-7-β-galactosylcoumarin, (*6*). Label (*6*) can be attached to amino-containing or amino-modified ligands

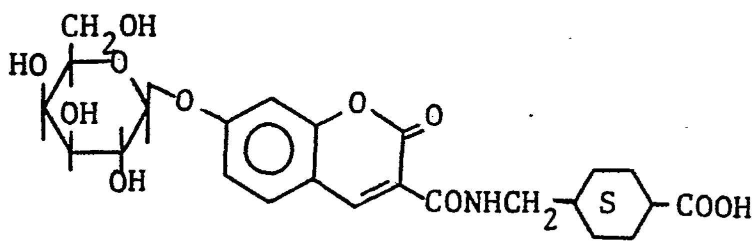

(*6*)

by appropriate amide and peptide bond forming reactions.

A particularly preferred rigid portion inserted into linking group R for the purposes of the present invention is N,N'-piperazinylene, yielding conjugates of the general formula:

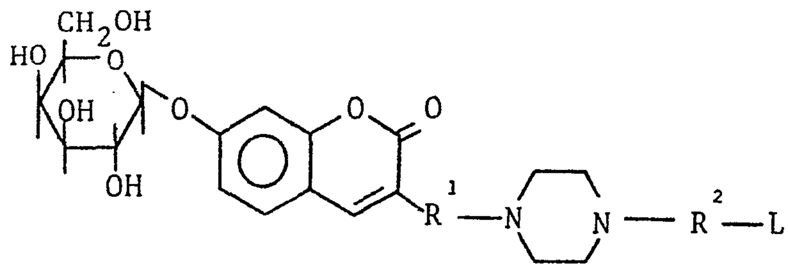

wherein $R^1$ and $R^2$ are appropriate bridging elements. Conjugates of such general type which have been found to be particularly useful are those of the following structure:

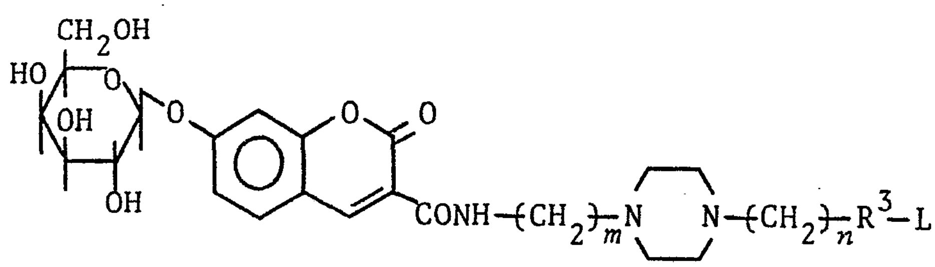

wherein *m* and *n* are integers as described in the examples which follow, $R^3$ is an appropriate bridging element, e.g., a bond or a chain of from 1-20 atoms, excluding hydrogen, and L is as above. Conjugates of this type are particularly exemplified in the experimental section which follows.

*2. Fluorescence quenching or enhancement*

The labeled conjugate in this system is composed, in its label portion, of a fluorescer whose fluorescence is quenched or enhanced in some measurable

degree when the labeled conjugate is bound by antibody. The fluorescent label is usually measured directly, with its fluorescence being the detectable signal. Assay systems of this type are described in U.S. Pat. Nos. 3,940,475; 4,150,949; 4,160,016, 4,160,818 and 4,272,505; in U.K. Pat. Spec. 1,583,869; and in *J. Clin. Path.* *30*:526(1977), as well as the other references cited hereinabove.

3. *Fluorescence polarization*

The label in this system is also a fluorescer; however, the affected characteristic is polarization of fluorescence due to binding of the labeled conjugate by antibody. Assay systems of this type are described in *J. Exp. Med.* *122*:1029(1975) and in the references cited hereinabove.

4. *Energy transfer*

In this system, the label is one member of an energy transfer donor-acceptor pair and the antibody is conjugated with the other of such pair. Thus, when the labeled conjugate is bound by antibody, the energy expression of the donor component of the pair is altered by transfer to the acceptor component. Usually, the donor is a fluorescer and the acceptor is a quencher therefor, which quencher may or may not be a fluorescer as well. In such embodiment, the detectable signal is fluorescence, but other detectant systems are possible also. Such assay systems are described in U.S. Pat. Nos. 3,996,345; 4,174,384; and 4,199,559 and in U.K. Pat. Spec. 2,018,424; as well as the references cited hereinabove.

*5. Chemically-excited fluorescence*

In this system, the label is again a fluorescer, however, the ability of the fluorescer label to be chemically excited to an energy state at which it fluoresces is affected by binding of the labeled conjugate with antibody. Chemical excitation of the label is usually accomplished by exposure of the fluorescer label to a high energy compound formed *in situ*. Assay systems of this type are described in commonly owned U.S. Pat. No. 4,238,195.

*6. Double antibody steric hindrance*

Another assay system is the double antibody immunoassay system described in U.S. Pat, Nos. 3,935,074 and 3,998,943. The labeled conjugate comprises two epitopes, one of which participates in the immunological reaction with the ligand and ligand antibody and the other of which is bindable by a second antibody, with the restriction that the two antibodies are hindered from binding to the labeled conjugate simultaneously. The second epitope can be a fluorescent substance whose fluorescence is quenched by the second antibody binding, or may participate in an ancillary competitive binding reaction with a labeled form of the second epitope for binding to the second antibody. Various detectant systems are possible in such a system as described in the aforementioned patents. Related assay systems are described in U.S. Pat. Nos. 4,130,462 and 4,161,515 and in U.K. Pat. Spec. 1,560,852; as well as the other references cited hereinabove.

*B. Heterogeneous Formats*

The present assay method can also be applied to the conventional heterogeneous type assay techniques wherein the bound- and free-species of the labeled conjugate are separated and the label component in one or the other is determined. The reagent means for performing such a heterogeneous assay can take many different forms. In general, such means comprises three basic constituents, which are (1) the analyte to be detected, (2) a binding counterpart of the analyte and (3) the labeled conjugate. The binding reaction constituents are combined simultaneously or in a series of additions, and with an appropriate incubation period or periods, the labeled conjugate becomes bound to its corresponding binding partners such that the extent of binding, i.e., the ratio of the amount of labeled conjugate bound to a binding counterpart (the bound-species) to that unbound (the free-species), is a function of the amount of analyte present. The bound- and free-species are physically separated and the amount of label present in one thereof is determined by exciting the photophor by appropriate means and measuring the resulting light emitted. The measured emission can be compared to a negative control or standard results, e.g., a standard curve.

Various means of performing the separation step and of forming the binding reaction systems are available in the art. Separation can be accomplished by such conventional techniques as those involving what is commonly known as a solid-phase antibody or antigen, a second antibody, or a solid-phase second antibody, as well as the use of immune complex precipitation agents, absorbents, and so forth. Binding reaction systems that can be followed include the so-called competitive binding technique, the sequential saturation technique, the "sandwich" technique, and so forth. Further details concerning the various known heterogeneous systems are readily available in the literature, e.g., U.S. Patent Nos. 3,720,760; 3,940,475; 3,992,631; 4,058,732; 4,133,639; 4,144,452; 4,171,311; 4,201,763; and 4,230,797; and *J. Pharm. Sci.* *70*:469(1981) and the articles cited therein.

It is contemplated that manipulative schemes involving other orders of addition and other binding reaction formats can be devised for carrying out homogeneous and heterogeneous specific binding assays without departing from the inventive concept embodied herein.

*REACTION MIXTURE AND CONDITIONS*

The test sample to be assayed can be a naturally occurring or artificially formed liquid suspected to contain the analyte, and usually is a biological fluid or a dilution thereof. Biological fluids that can be assayed include serum, plasma, urine, saliva, milk, and amniotic and cerebrospinal fluids.

The binding reaction will in almost all cases be allowed to proceed under mild conditions. The reaction mixture will be in general an aqueous medium with any desirable organic cosolvents being present in minor amounts. The temperature of the reaction will be maintained at a constant level in normal circumstances throughout the incubation period and the measurement step. Temperatures will generally be between 5 and 50°C, more usually between 20 and 40°C. Preferably, the reaction will proceed at room temperature. The pH of the reaction mixture will vary between 5 and 10, more usually between 6 and 9. The concentration of various reagents will depend on the level of analyte expected in the test medium, with such level usually being between $10^{-3}$ and $10^{-12}$M. As in the case of the previously described reaction parameters, selection is primarily based on empirically derived optimization balanced against the preferences and needs of the technician who will ultimately perform assays on a routine basis. None of the parameters therefore is of a critical nature to the present invention, rather thay are all within the ordinary skill in the art.

*REAGENT SYSTEM*

The reagent system, i.e., reagent combination or means, of the present invention comprises all of the essential chemical elements required to conduct a desired assay method encompassed by the present invention. The reagent system is presented in a commercially packaged form, as a composition or admixture where the compatibility of the reagents will allow, in a test device configuration, or as a test kit, i.e., a packaged combination of one or more containers holding the necessary reagents. Included in the reagent system are the reagents appropriate for the binding reaction system desired, always requiring a labeled conjugate and the analyte binding partner as defined hereinbefore. Such binding reaction reagents can include, in addition to the labeled conjugate and the binding counterpart to the analyte, any other necessary or optional reagents for performing the particular assay technique involved, e.g., the enzyme required when using an enzyme substrate-labeled technique. Of course, the reagent system can include other reagents as are known in the art and which may be desirable from a commercial and user standpoint, such as buffers, diluents, standards, and so forth. Also preferred is a test device comprising the reagent system and a solid carrier member incorporated therewith. The various forms of such test device are described in application Serial No. 202,378, filed October 30, 1980, which is incorporated herein by reference.

The present invention will now be illustrated, but is not intended to be limited, by the following examples.

EXAMPLE 1

In this example, a water soluble linking group containing a rigid N,N'-piperazinylene portion was used to hinder molecular stacking in a bichromophoric system consisting of an umbelliferone derivative as a fluorescer label and quinidine (6'-methoxycinchonine) as a quenching analyte. These two aromatic residues were linked through a flexible alkyl chain of varying lengths, namely 4 and 12 methylene groups (*I* and *II* in Fig. 1). To incorporate a degree of rigidity to the linking group, conjugates were also prepared where the linking group included a rigid piperazinylene residue (*III* and *IV* in Fig. 1)

A. Preparation of conjugates

β-Galactosyl-umbelliferone-quinidine, *I*.

The structure of this compound is shown in Fig. 1 of the drawings.

A mixture of 6.6 grams (g) [21 mmol (millimoles)] of 6'-hydroxycinchonine [Small et al, *J. Med. Chem. 22*: 1014(1979)], 7 g (25 mmol) of N-(4-bromobutyl) phthalimide (Aldrich Chemical Co., Milwaukee, WI), 3.45 g (25 mmol) of potassium carbonate [$K_2CO_3$], 100 milligrams (mg) of 18-Crown-6 (1,4,7,10,13,16-hexaoxacyclooctadecane, Aldrich), and 150 milliliters (ml) of acetone was refluxed under argon for 16 hours. Work up by chromatography on silica gel, followed by recrystallization from methanol gave 3.1 g of 6'-(4-phthalimidobutoxy)cinchonine as white crystals, mp 228°C.

This phthalimido derivative, 3 g (5.9 mmol), 5 ml of hydrazine, and 100 ml of methanol were heated for 1 hour, then cooled and solvent removed. The solid residue was stirred with 100 ml of 1 N hydrochloric acid and filtered to remove phthalhydrazide. Neutralization of the filtrate with sodium hydroxide produced a precipitate which was recrystallized from aqueous methanol to give 1.6 g of 6'-(4-aminobutoxy)cinchonine as white needles, mp 126-128°C.

Analysis: Calcd. for $C_{23}H_{31}N_3O_2$: C, 72.51; H, 8.19; N, 11.01
Found: C, 72.97; H, 8.33; N, 10.77

A solution of 434 mg (1.2 mmol) of 7-β-galactosylcoumarin-3-carboxylic acid [Burd et al, *Clin. Chem.* *23*:1402(1977)] in 20 ml dry dimethylformamide (DMF) was cooled to -10°C and combined with 120 mg (1.2 mmol) of triethylamine and 160 mg (1.2 mmol) of isobutyl chloroformate. After 10 minutes, the white precipitate of triethylamine hydrochloride was removed by filtration and 450 mg (1.2 mmol) of 6'-(4-aminobutoxy)cinchonine was added. After stirring 1 hour at room temperature the solvent was removed and the residue chromatographed on silica gel. The column was eluted with 3:2 v/v ethyl acetate:ethanol and 15 ml fractions were collected. Fractions 44 through 80 were pooled and evaporated. Recrystallization from 2-propanol gave 279 mg of (*I*) as a fine white powder, mp 191-193°C.

Analysis: Mass Spectrum (field desorption):
m/e 713 [$M^+$ minus $H_2O$];
551 [$M^+$ minus $H_2O$, minus $C_6H_{10}O_5$].

β-Galactosyl-umbelliferone-quinidine, *II*.

The structure of this compound is shown in Fig. 1 of the drawings.

A mixture of 7.76 g (25 mmol) of 6'-hydroxycinchonine, 11.83 g (30 mmol) of N-(12-bromododecyl) phthalimide (Aldrich), 4.14 g of $K_2CO_3$, and 75 ml of dry acetone was stirred at reflux under argon for 16 hours. The reaction was cooled and worked up as before to give 7 g of 6'-(12-phthalimidododecyloxy) cinchonine as an off-white solid, mp 232-234°C (decomposed).

A solution of 1.57 g (25 mmol) of this phthalimide and 2.13 ml of hydrazine in 50 ml of methanol was refluxed 1 hour. Volatile material was removed on the rotary evaporator and the solid residue dried under high vacuum overnight. This gave 6'-(12-aminododecyloxy)cinchonine as a gray solid which was not further characterized. One mmol was reacted with 7-β-galactosylcoumarin-3-carboxylic acid in a manner similar to the previous example. The reaction product was purified by silica gel chromatography and recrystallization from ethanol to give 70 mg of (*II*) as tan crystals, mp 118°C.

Analysis: Calculated for $C_{47}H_{61}N_3O_{11}$: C, 66,88; H, 7.29; N, 4.98.
Found: C, 66.75; H, 7.31; N, 4.63.

β-Galactosyl-umbelliferone-piperazinyl-quinidine, *III*.

The structure of this compound is shown in Fig. 1 of the drawings.

1-(2-Aminoethyl)piperazine (Aldrich) was converted to the N-trifluoroacetamide derivative by treatment with ethyl trifluoroacetate. Recrystallization from toluene gave white needles, mp 92-93°C.

Analysis: Calculated for $C_8H_{14}F_3N_3O$: C, 42.66; H, 6.25; N, 18.66.
Found: C, 42.68; H, 5.85; N, 19.12.

Ten grams (44 mmol) of this substance was allowed to stand for 16 hours in 50 ml of methanol containing 2.9 g of ethylene oxide and 1 ml of glacial acetic acid. Removal of solvent and recrystallization from toluene gave 10.1 g of 1-(2-hydroxyethyl)-4-(2-trifluoroacetamidoethyl)piperazine (the hydroxy derivative) as white needles mp 84°C.

Analysis: Calculated for $C_{10}H_{18}F_3N_3O_2$: C, 44.61; H, 6.74; N, 15.61.
Found: C, 45.24; H, 6.72; N, 15.59.

The hydroxy derivative (3 g, 11 mmol) and 1.33 g (13 mmol) of triethylamine were dissolved in 50 ml of methylene chloride and cooled to 5°C while stirring under argon. To this was added 1 equivalent of methanesulfonyl chloride, followed 1 hour later by 3.4 g (11 mmol) of 6'-hydroxycinchonine and 4.5 g (24.5 mmol) of anhydrous $K_2CO_3$. The reaction was allowed to warm to room temperature and stir for 17 hours. It was filtered, evaporated and the residue

heated in methanolic potassium hydroxide for 4 hours to remove the trifluoroacetyl group. The product was chromatographed on silica gel eluting with 4:4:1 v/v/v chloroform:methanol:concentrated ammonium hydroxide to give 2.4 g of 6'-[[2-[4-(2-aminoethyl) piperazinyl-1]ethoxy]]cinchonine as a brown amorphous solid which was homogeneous on thin layer chromatography (tlc) and ninhydrin-positive. This amine was not further characterized. One mmol was reacted with 7-β-galactosylcoumarin-3-carboxylic acid (*supra*) in a manner similar to the previous example. The reaction product was chromatographed on silica gel, eluting with 4:1 v/v ethanol:1 M aqueous triethylammonium bicarbonate, then on Sephadex LH-20 eluting with methanol. This gave 343 mg of (*III*) as a tan amorphous solid.

Analysis: Calculated for $C_{43}H_{53}N_5O_{11} \cdot 2H_2O$:
C, 60.62; H, 6.74; N, 8.22.
Found: C, 59.43; H, 6.99; N, 7.99.

* * * * * *

The above described synthesis of the β-galactosyl-umbelliferone-piperazinyl-quinidine conjugate (*III*) can be modified to yield conjugates wherein, independently, *m* and *n* = 2 through 9 by replacing 1-(2-hydroxyethyl)-4-(2-trifluoroacetamidoethyl) piperazine with the appropriate synthetic intermediate represented by general formula *C* in Diagram 1, where G represents an appropriate amine protecting group. To obtain intermediates such as *C*, piperazine can be reductively alkylated with 1 equivalent of a suitably protected β-aminoaldehyde *A*. The resulting mono-

Diagram 1

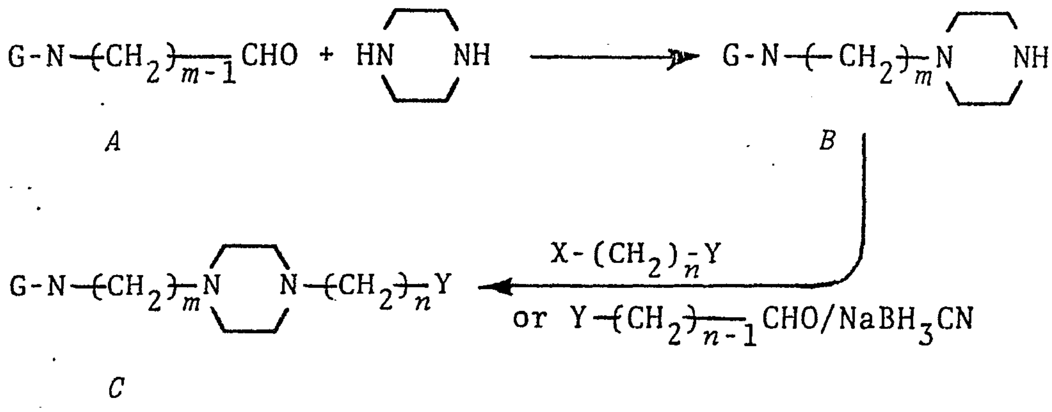


A B C

substituted piperazine *B* can be converted to intermediate *C* by common organic chain-forming reactions. For example, *B* can be alkylated with the species $X-(CH_2)_n-Y$ where X is a leaving group (such as bromo, chloro, *p*-toluene sulfonyl, methanesulfonyl) and where Y is either itself a leaving group or a functional group such as hydroxyl or acetoxy which can, in one or more steps, be transformed into a leaving group. Bi-functional intermediates such as $X-(CH_2)_n-Y$ are well-known in organic chemistry, being obtainable by simple chemical transformations from the class of *alpha, omega*-dihydroxyalkanes or the *alpha, omega*-halohydrins. (c.f., "Chem. Sources USA", 1979 ed., Directories Publishing Co., Flemington, NJ)

In general, alkylation of mono-substituted piperazines *B* with reactants $X-(CH_2)_n-Y$ where both X and Y are leaving groups will be less preferred because of the necessity of closely controlling reaction conditions in order to avoid over-alkylation of the piperazine ring.

The transformation of *B* to *C* can also be achieved by the reductive alkylation of *B* with *omega*-substituted

aldehydes $Y-(CH_2)_{n-1}CHO$ [Borch *et al.*, *J. Amer. Chem. Soc.* *93*:2897(1971)] where Y is a leaving group as defined above, or a functional group such as hydroxyl or acetoxy which can be transformed into a leaving group in one or more steps. Reactants such as $Y-(CH_2)_{n-1}CHO$ are available from the class of *omega*-hydroxy aldehydes [c.f., Hurd and Saunders, Jr., *J. Amer. Chem. Soc.* *74*:5324(1952)].

*Omega*-Aminoaldehydes $NH_2-(CH_2)_{m-1}CHO$ where *m* = 2 through 5 are known compounds ("Chemistry of Carbon Compounds", Rodd, ed., Vol. I, Elsevier Publ. Co., New York, 1951, p. 706) During the reductive alkylation reaction [Borch et al, *supra*] the amino group will be protected with a group G which is stable under the reaction conditions but which can be removed under conditions not harmful to the molecule. A large number of blocking groups G are known which can be removed under acidic or basic conditions, by catalytic hydrogenation, or by treatment with specific reagents (c.f., Greene, "Protective Groups in Organic Chemistry" John Wiley & Sons, New York, 1981, pp. 218-287). Higher members of the series (*m* = 6 through 9) can be prepared in protected form by known transformations of the previously mentioned *omega*-hydroxyaldehydes, $HO-(CH_2)_{m-1}CHO$. These substances can be converted to their acetals $HO-(CH_2)_{m-1}CH(OC_2H_5)_2$ as described in the above-cited reference Hurd and Daunders and the hydroxyl function transformed, in one or more steps, into an amino or protected amino function [c.f., Mitsunoku *et al*, *J. Amer. Chem. Soc.* *94*:679(1972)].

β-Galactosyl-umbelliferone-piperazinyl-quinidine, *IV*.

The structure of this compound is shown in Fig. 1 of the drawings.

A mixture of 5.8 g (19 mmol) of 6'-hydroxycinchonine, 3.87 g (19 mmol) of ethyl 5-bromopentanoate, 1.87 g of $K_2CO_3$, 100 mg of 18-crown-6, and 150 ml of acetone was refluxed under argon for 16 hours. Solvent was removed and the residue partitioned between methylene chloride and dilute sodium hydroxide (NaOH). The organic phase was separated, dried and evaporated, yielding 2.6 g of the ethyl ester as an orange solid. It was refluxed overnight in 125 ml of methanol containing 1.25 g $K_2CO_3$. The methanol was removed and the residue dissolved in $H_2O$. A precipitate occured when the pH was adjusted to 7 with dilute hydrochloric acid (HCl). Recrystallization from aqueous ethanol gave 1.6 g of 6'-(4-carboxybutoxy)cinchonine (the acid) as an off-white solid, mp 148-153°C.

Analysis: Calculated for $C_{24}H_{30}N_2O_4 \cdot H_2O$: C, 67.27; H, 7.53; N, 6.54.
Found: C, 67.42; H, 7.59; N, 6.59.

A solution of 100 g (0.42 mol) of *tert*-butyl S-(4,6-dimethylpyrimidin-2-yl)thiolcarbonate (Aldrich) in 400 ml of dry dioxane was added at room temperature, dropwise over a period of 6 hours, to a solution of 182 g (0.91 mol) of 1,4-*bis*-(3-aminopropyl)piperazine (Aldrich) in 360 ml of dioxane. After stirring overnight the reaction was filtered and evaporated to give an oily residue. It was dissolved in 2 liters (L) of distilled $H_2O$ and applied to a 5 cm x 90 cm

column of Amberlite IRC-50 ion exchange resin ($NH_4^+$ form) (Rohm and Haas, Philadelphia, PA). The column was washed with 500 ml of $H_2O$, then eluted with a linear gradient of 2 L $H_2O$ to 2 L of 0.5 M ammonium hydroxide. The desired fractions were combined and evaporated to give 65 g of 1-(3-aminopropyl)-4-(3-*tert*-butyloxycarbonyl-aminopropyl)piperazine (the first amine) as a light yellow oil.

Analysis: Mass Spectrum (70 e.V.): m/e 301 [$MH^+$]; 201 [$MH^+$ minus $COOC(CH_3)_3$].

A solution of the acid (*supra*) (2.05 g, 5 mmol) and 1.65 g (10 mmol) of N-hydroxysuccinimide in 25 ml of dry DMF was cooled to 0°C while stirring under argon and 1.13 g (5.5 mmol) of dicyclohexylcarbodiimide added. The cooling bath was removed and the reaction stirred at room temperature for 3 hours; after which 1.5 g (5 mmol) of the first amine (*supra*) was added. After stirring overnight at room temperature, solvent was removed and the residue chromatographed on silica gel eluting with 7:3 v/v ethanol:1 M aqueous triethyl-ammonium bicarbonate. This gave 1.5 g of product as a tan, glassy solid.

Analysis: Calculated for $C_{39}H_{60}N_6O_5 \cdot H_2O$: C, 65.88; H, 8.79; N, 11.82.
Found: C, 65,32; H, 8.83; N, 12.18.

This substance (1.5 g, 2.2 mmol) was stirred for 3 hours at -10°C in trifluoroacetic acid to remove the *tert*-butyloxycarbonyl blocking group. The resulting second amine was not purified further. It was taken up in 50 ml of $H_2O$ and the pH adjusted to 8.0 with concentrated NaOH solution.

A second solution was prepared by dissolving 800 mg (2.2 mmol) of 7-β-galactosylcoumarin-3-carboxylic acid (*supra*) and 500 mg (5.3 mmol) of N-hydroxysuccinimide in 10 ml of DMF. The solution was cooled to 0°C while stirring under argon and 489 mg (2.4 mmol) of dicyclohexylcarbodiimide was added. The cooling bath was removed, and the reaction allowed to warm to room temperature and stir for 2 hours to generate the N-hydroxysuccinimide ester.

The pH 8 aqueous solution of the second amine (*supra*) was cooled to 0°C and to it was added the DMF solution of the activated ester dropwise over 5 minutes, followed by an additional 10 ml of DMF, all while keeping the temperature below 5°C. The pH of the final solution was adjusted to 7.0 with sodium hydroxide and the reaction allowed to stir at room temperature for 4 hours. The crude product was chromatographed on 180 g of silicic acid eluting with a linear gradient of 2 L of ethanol to 2 L of 7:3 v/v ethanol:1 M aqueous triethylammonium bicarbonate. This gave 900 mg of the desired product as a glassy solid. Three hundred mg of it was chromatographed on a 90 cm by 2.5 cm column of Sephadex LH-20 eluting with methanol. This gave 160 mg of (*IV*) as a pale-tan, glassy solid.

Analysis: Calculated for $C_{50}H_{66}N_6O_{12}$: C, 63,68; H, 7.05; H, 8.91.

Found: C, 63.53; H, 6.82; N, 8.65.

* * * * *

The above described synthesis of the β-galactosyl-umbelliferone-piperazinyl-quinidine conjugate (*IV*) can be modified to yield conjugates where $r = 1$ through 9 by replacing ethyl 5-bromobutyrate in the synthesis of *IV* with the appropriate ethyl *omega*-chloro- or bromoalkanoate $X-(CH_2)_r-COOC_2H_5$. Examples having $r = 1$ through 4 are commercially available; $r = 5$ through 9 are described in Barger *et al*, *J. Chem. Soc. 1937*:714, Salmon-Legagneur, *Bull. Soc. Chem. France 1956*:411; and Linnell and Vora, *J. Pharm. Pharmacol. 4*:55(1952).

Conjugates wherein $n = 2$ through 9 and $m = 2$ through 9 can be prepared by replacing 1-(3-aminopropyl)-4-(3-*tert*-butyloxycarbonylamino-propyl) piperazine in the synthesis of *IV* with the appropriate 1,4-disubstituted piperazine *G* [see Diagram 2] Intermediates such as *G* can be obtained by reductively alkylating piperazine with 1 equivalent of a

DIAGRAM 2

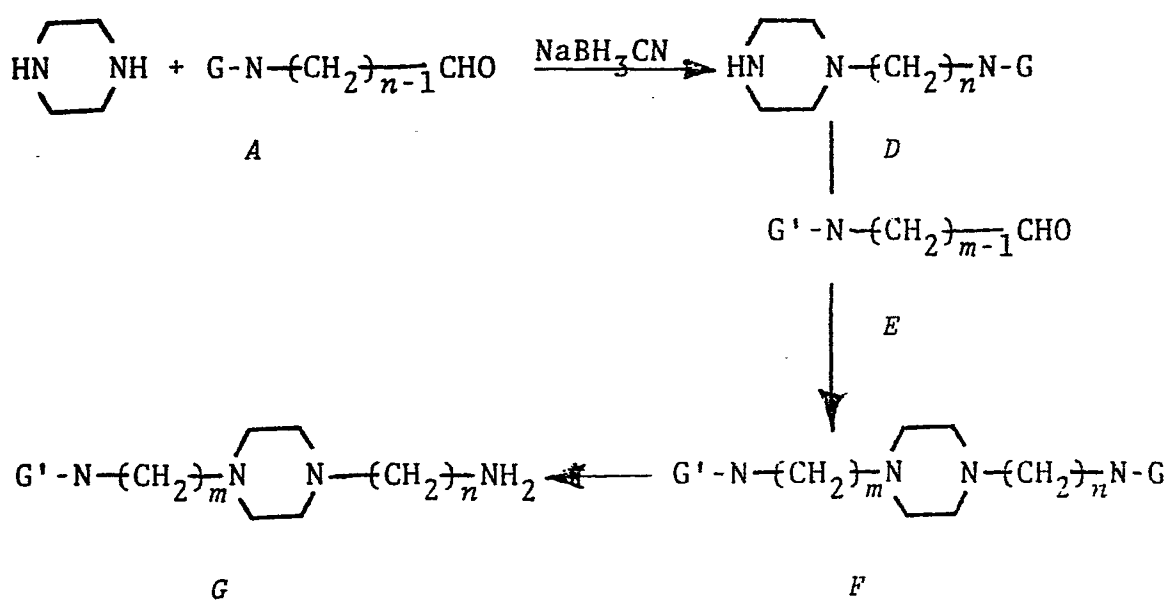

suitably protected *omega*-aminoaldehyde *A*. The resulting mono-substituted piperazine *D* can then be converted to the 1,4-disubstituted species *F* by alkylation with a second, protected *omega*-aminoaldehyde *E*. Where $m = n$, G and G' may be identical. Where $m \neq n$, blocking groups G and G' must be chosen so that one may be selectively removed in the presence of the other in order to generate intermediate *F*. A large number of these are known which can be removed under acidic or basic conditions, by catalytic hydrogenation, or by specific reagents such as fluoride ion, hydrazine, etc (c.f., Greene, *supra*).

B. Relative fluorescence and fluorescence lifetime studies.

Relative fluorescence intensity was determined on an SLM 8000 spectrofluorometer (SLM Instruments, Inc., Urbana, IL) with excitation wavelength set at 405 nanometers (nm) and emission wavelength set at 445 nm. Fluorescence lifetimes were determined by means of the cross-correlation phase and modulation fluorometer described by Spencer and Weber, *Ann. N.Y. Acad. Sci. 158*: 361(1969).

The fluorescence of a population of fluorescent molecules is affected (1) by the static quenching due to molecular interactions in the ground state before excitation and (2) dynamic quenching of the excited singlet state due to interactions throughout the excited singlet lifetime. Measurements of lifetimes and fluorescence in the absence and in the presence of quencher would yield $\gamma$, the degree of dissociation of the nonfluorescent complex at the time of the excitation since $\gamma = (\tau_o/\tau)/F_o/F)$ where $\tau_o$ and $F_o$ are the lifetime and fluorescence in the absence of quencher and $\tau$ and F in the presence of quencher.

The dynamic Stern-Volmer rate constant, $k_{+}^{*}$, which is the bimolecular rate constant for the quenching encounter when the fluorophore is in the excited state, is given by $k_{+}^{*} = (1/\tau - 1/\tau_{o})\ sec^{-1}$ while the equilibrium constant of association for complex formation in the ground state, $K_a$ is given by $K_a = (1/\gamma - 1)$. [Spencer and Weber in *Structure and Function of Oxidation Reduction Enzymes*, ed. Akeson et al, Pergamon Press (New York 1972) pp. 393-399].

The results are shown in Table A. The fluorescence data was collected for the fluorescent umbelliferone-quinidine products of β-galactosidase cleavage of the four labeled conjugates (*I-IV*) shown in Fig. 1 and for a fluorescent umbelliferone derivative (designated "RAS") that was unconjugated to analyte.

TABLE A

| Compounds | Linking arms | Relative Fluorescence | Lifetime (nano sec) | % in stacked form 100%-γ% | $K_a$ | $K_{\dagger}$* |
|---|---|---|---|---|---|---|
| RAS[1] | None | 100% | 3.1 | - | - | - |
| *I* | $-(CH_2)_4-$ | 5.2% | 2.3 | 93 | 13.05 | $1.2x10^5 sec^{-1}$ |
| *II* | $-(CH_2)_{12}-$ | 4.3% | 2.6 | 95 | 18.43 | $0.6x10^5 sec^{-1}$ |
| *III* | 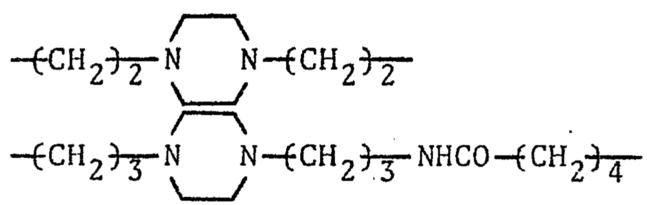 | 42.4 | 2.3 | 42 | 0.71 | $1.2x10^5 sec^{-1}$ |
| *IV* | | 31.2% | 2.3 | 58 | 1.35 | $1.2x10^5 sec^{-1}$ |

(1) N-(2-Hydroxyethyl)-7-hydroxycoumarin-3-carboxamide
(U.S. Pat. No. 4,273,715)

The relative quantum efficiencies for the umbelliferone fluorescence in the umbelliferone derivative and in the 4 umbelliferone-quinidine compounds indicate that the umbelliferone fluorescence in the umbelliferone-quinidine compounds are quenched to different extent (Table A). Compounds *I* and *II* only exhibit 5.2% and 4.3%, respectively, of the expected umbelliferone fluorescence, while Compounds *III* and *IV* exhibit 42.4% and 31.2%, respectively. The lifetimes for the umbelliferone fluorescence in the umbelliferone derivative and in the four umbelliferone-quinidine compounds are also shown in Table A.

Further, the Table A data, which lists the calculated kinetic and equilibrium parameters, indicates that:

(a) At 26°C in Bicine buffer, pH 8.5, the umbelliferone-quinidine compounds with an alkyl chain of 4 and 12 methylene groups exist mainly (93% and 95% in *I* and *II*, respectively) as an internally stacked form. Introduction of a rigid piperazine linking arm reduces the stacked form to 42% and 58% in *III* and *IV* with 4 and 10 methylene groups, respectively.

(b) The intramolecular interaction between umbelliferone and quinidine is stronger when the linkage is flexible ($K_a$ = 0.7 in *III* and 1.4 in *IV*). The 14- and 19-fold decrease in the equilibrium constant of association between umbelliferone and quinidine, when the rigid piperazine linking arm is introduced, is concluded to be due to the destabilizing effect of the rigid arm.

(c) Although the dynamic rate constants for the 4 umbelliferone-quinidine compounds ranging from $0.6 \times 10^5$ to $1.2 \times 10^5$ $sec^{-1}$ are low, they are as expected nearly identical because dynamic quenching is solely a diffusion phenomenon.

C. Quinidine immunoassay

A substrate-labeled fluorescent immunoassay (SLFIA) for quinidine was performed as follows:

(1) Reagents

A. Antibody/Enzyme Reagent - 50 mM Bicine buffer (Calbiochem-Behring, La Jolla, CA), pH 8.5, containing 5 Units/liter β-galactosidase, sufficient antiserum raised against a quinidine immunogen to decrease fluorescence to 20% of that in the absence of antiserum, and 15.4 mM sodium azide.

B. Conjugate Reagent - 5 mM formate buffer, pH 3.5, containing 0.92 mM β-galactosyl-umbelliferone-quinidine (*IV*), 0.01% Tween 20 (Atlas Chemical Co.) and 15.4 mM sodium azide.

C. Quinidine Standards - USP-NF Reference standard quinidine gluconate added to normal human serum, diluted 51-fold with 50 mM Bicine buffer, containing 15.4 mM sodium azide.

(2) Assay protocol

To 3 ml volumes of the Antibody/Enzyme Reagent in cuvettes were added 100 µl of the diluted quinidine standards. To begin the reaction, 100 µl of the Conjugate Reagent was added to each cuvette with mixing. After 20 minutes the fluorescence intensity was measured in each cuvette (excitation 400 nm, emission 450 nm).

(3) Results

Performance of the assay yielded the following results:

| Quinidine (µg/ml) | Fluorescence Units |
|---|---|
| 0 | 29.2 |
| 2.5 | 42.0 |
| 5.0 | 61.0 |
| 7.5 | 77.6 |
| 10.0 | 90.0 |

EXAMPLE 2

In this example, another bichromophoric system was studied using linking groups in the labeled conjugate which had varied rigidity. This system consisted of an umbelliferone derivative as a fluorescer label and thyroxine as a quenching analyte. The fluorescer and analyte were linked through flexible groupings (*V*, *VI*, and *VII* in Fig. 2) and through rigid groupings (*VIII* and *IX* in Fig. 2a).

A. Preparation of conjugates

β-Galactosyl-umbelliferone-thyroxine, *V*.

The structure of this compound is shown in Fig. 2 of the drawings.

To a mixture of 17.74 g (0.1 mol) of 4-aminobutyraldehyde diethylacetal (Aldrich), 22.1 ml (0.16 mol) of triethylamine, and 100 ml of dry ethanol at 0°C was added 21.3 g (0.16 mol) of ethyl trifluoroacetate over 15 min.

The reaction was allowed to warm to room temperature and stir overnight. Solvent was removed under reduced pressure, the residue taken up in ether, and washed with brine. The organic phase was dried, filtered, and evaporated leaving a brown oil which was purified by fractional distillation to give 24.6 g of 4-(trifluoroacetamido)butyraldehyde diethylacetal as a colorless oil, bp 104-105°C (0.01 torr).

Analysis: $^1$H NMR Spectrum ($CDCl_3$): 1.2 (t, J=7 Hz, 6H); 4.3 (m, 4H); 3.2-4.0 (m, 6H); 4.5 (t, J=5 Hz, 1H); 6.3 (s, 1 H).
Infrared Spectrum (neat): 1705 $cm^{-1}$ (CO).

A solution of 3.1 g (12 mmol) of this acetal in 30 ml of tetrahydrofuran (THF):acetic acid:$H_2O$ (1:1:1) was stirred at room temperature under argon for 48 hours. Evaporation gave an oil which was combined with 10.1 g (12 mmol) of thyroxine ethyl ester [Clayton and Hems, *J. Chem. Soc.* *1950*:840] and 275 ml of absolute ethanol. Sodium cyanoborohydride (422 mg, 6.8 mmol) was added and the mixture stirred for 24 hours at room temperature. The reaction was filtered, evaporated under

reduced pressure, and the residue recrystallized from methylene chloride:ethyl acetate to give 4.32 g of N-(4-trifluoroacetamidobutyl)thyroxine ethyl ester as a white powder, mp 202-204°C.

Analysis: Calculated for $C_{23}H_{22}I_4F_3N_2O_5 \cdot 2H_2O$:
C, 27.40; H, 2.70; N, 2.78.
Found: C, 27.36; H, 2.45; N, 2.77.

A solution of 2.5 g (2.6 mmol) of N-(4-trifluoroacetamidobutyl)thyroxine ethyl ester in 100 ml of anhydrous ethanol was heated to reflux and treated with hydrogen chloride gas for 7 hours. The reaction was cooled, concentrated under reduced pressure, and the residue twice precipitated from ethanol-ether to give 2.1 g of N-(4-aminobutyl) thyroxine ethyl ester dihydrochloride (the dihydrochloride) as an off-white powder which analyzed as the *bis*-ethanolate.

Analysis: Calculated for $C_{21}H_{26}I_4Cl_2N_2O_4 \cdot 2(C_2H_6O)$:
C, 28.84; H, 3.67; N, 2.69.
Found: C, 29.32: H, 3.28; N, 2.91.

To 184 mg (0.5 mmol) of 7-β-galactosylcoumarin-3-carboxylic acid, *supra*, and 64 mg (0.55 mmol) of N-hydroxysuccinimide in 3 ml of dry DMF at 0°C was added 108 mg (0.5 mmol) of dicyclohexylcarbodiimide and 0.75 ml of DMF. The reaction was allowed to warm to room temperature and stir 90 minutes, then combined with a second solution prepared by dissolving 521 mg (0.5 mmol) of the dihydrochloride in 5 ml of dimethylsulfoxide (DMSO) containing 0.350 ml (5 equiv.) of

triethylamine. After stirring for 24 hours at room temperature, solvent was removed and the residue chromatographed on 70 g of silicic acid eluting with a linear gradient of ethyl acetate to 1:1 v/v ethyl acetate:ethanol. Recrystallization from ethanol gave 318 mg of the ethyl ester of N-[4-(7-β-galactosylcoumarin-3-carboxamido)butyl]thyroxine (the ethyl ester) as a white powder, mp 118°C.

Analysis: Calculated for $C_{37}H_{38}I_4N_2O_{13}$: C, 36.24; H, 3.12; N, 1.96.
Found: C, 36.02; H, 2.97; N, 1.90.

To 569 mg (0.5 mmol) of the ethyl ester was added 23 ml of 7% $K_2CO_3$ solution in aqueous methanol. After 21 hours stirring at room temperature, a clear solution was obtained. It was diluted to 60 ml with $H_2O$ and the pH adjusted to 6.0 with dilute acetic acid. This gave a precipitate which was washed with hot methanol to give 489 mg of N-[4-(7-β-galactosylcoumarin-3-carboxamido)butyl]thyroxine (*V*) as a white solid, mp 186°C.

Analysis: Calculated for $C_{35}H_{34}I_4N_2O_{13}$: C, 35.08; H, 2.86; N, 2.34.
Found: C, 34.68; H, 2.94; N, 2.91.

<u>β-Galactosyl-umbelliferone-thyroxine Ethyl Ester, *VI*.</u>

The structure of this compound is shown in Fig. 2 of the drawings.

A solution of 366 mg (1 mmol) of 7-β-galactosylcoumarin-3-carboxylic acid, *supra*, 115 mg (1 mmol) of N-hydroxysuccinimide, and 206 mg (1 mmol) of dicyclohexylcarbodiimide in 20 ml of DMF was stirred for 1 hour at room temperature. To this was added 805 mg (1 mmol) of thyroxine ethyl ester and the reaction

allowed to stir overnight. Solvent was removed and the crude product chromatographed on 250 g of silica gel eluting with ethyl acetate:ethanol. Recrystallization from ethanol gave 340 mg of (*VI*) as a white solid, mp 187-188°C.

Analysis: Calculated for $C_{33}H_{29}I_4NO_{13}$: C, 34.31; H, 2.53; N, 1.21.
Found: C, 34.77; H, 2.87; N, 1.17.

β-Galactosyl-umbelliferone-thyroxine Ethyl Ester, *VII*.

The structure of this compound is shown in Fig. 2 of the drawings.

A solution of 3.67 g (10 mmol) of 7-β-galactosylcoumarin-3-carboxylic acid, *supra*, 1.05 g (10 mmol) of N-hydroxysuccinimide, and 2.06 g (10 mmol) of dicyclohexylcarbodiimide in 100 ml of DMF was stirred at room temperature for 2 hours. It was filtered and the filtrate added dropwise over a 15 minute period to a 0°C solution of 1.45 g (10 mmol) of 6-aminohexanoic acid and 2.02 g (20 mmol) of triethylamine in 100 ml of $H_2O$. After 2 hours solvent was removed and the residual oil chromatographed on silicic acid, eluting with ethyl acetate:ethanol mixtures. Recrystallization from ethanol gave 2 g of N-(5-carboxypentyl)-7-β-galactosylcoumarin-3-carboxamide as pale yellow crystals, mp 170°C.

Analysis: Calculated for $C_{22}H_{27}NO_{11}$: C, 54.88; H, 5.65; N, 2.91.
Found: C, 54.27; H, 5.65; N, 2.91.

A mixture of 481 mg (1 mmol) of this acid, 101 mg (1 mmol) of triethylamine, and 10 ml of DMF was cooled to -10°C while stirring under argon. To this was added 136 mg of isobutyl chloroformate followed 15 minutes later by 885 mg (1.1 mmol) of thyroxine ethyl ester. The reaction was stirred at -10°C for 1 hour, then allowed to warm to room temperature overnight. Solvent was removed and the residue chromatographed on silica gel eluting with ethyl acetate-ethanol mixtures. Recrystallization from methanol gave 400 mg of (*VII*) as a white solid, mp 148°C.

Analysis: Calculated for $C_{39}H_{40}I_4N_2O_{12}$: C, 36.93; H, 3.18; N, 2.21.
Found: C, 37.61; H, 3.55; N, 2.13.

β-Galactosyl-umbelliferone-piperazinyl-thyroxine, *VIII*.

The structure of this compound is shown in Fig. 2a of the drawings.

1-(2-Trifluoroacetamidoethyl)piperazine (56 g, 0.25 mol,) 37.5 ml of triethylamine, and 30.3 g (0.27 mol) of ethyl chloroacetate were combined in 250 ml of DMF. The temperature rose to 70°C in 5 minutes. The reaction was allowed to stir overnight at room temperature, then filtered to remove triethylammonium hydrochloride. The solvent was removed leaving an oily residue which was taken up in 250 ml of chloroform and washed with two 300 ml portions of $H_2O$. The organic phase was dried and evaporated to give 51 g of ethyl 4-(2-trifluoroacetamidoethyl)piperazine-1-acetate as a tan solid. This was not further characterized but was stirred with 20 g of potassium hydroxide in 250 ml of 4:1 methanol:$H_2O$ for 6 hours. The product was chromatographed on 1400 g of silica gel eluting with 4:4:1 v/v/v chloroform, methanol, concentrated ammonium hydroxide. Recrystallization from

methanol gave 22.5 g of 4-(2-aminoethyl)piperazine-1-acetic acid as white crystals, mp 225°C (dec).

A mixture of 2.6 g (6.5 mmol) of 7-β-galactosylcoumarin-3-carboxylic acid, *supra*, 730 mg (6.5 mmol) of N-hydroxysuccinimide, 1.3 g (6.3 mmol) of dicyclohexylcarbodiimide in 40 ml of DMF was stirred at room temperature for 36 hours. It was filtered to remove the precipitated dicyclohexyl urea, and the filtrate added dropwise to a cold solution of 1.2 g (6.4 mmol) of 4-(2-aminoethyl)piperazine-1-acetic acid in 40 ml of $H_2O$. To this was added 520 mg (6.2 mmol) of $NaHCO_3$ and the reaction allowed to warm to room temperature overnight. The precipitate which formed was recrystallized from $H_2O$:2-propanol giving 2.2 g of 4-[2-(7-β-galactosylcoumarin-3-carboxamido) ethyl]piperazine-1-acetic acid (the label acid derivative) as a white solid. A 250 mg sample was recrystallized from $H_2O$:methanol yielding 180 mg of white crystals, mp 187-192°C.

Analysis: Calculated for $C_{24}H_{31}O_{11} \cdot H_2O$: C, 51.89; H, 5.98; N, 7.46.

Found: C, 51.87; H, 5.90; N, 7.90.

N-(4-Trifluoroacetamidobutyl)thyroxine ethyl ester, *supra*, (2.0 g, 2.1 mmol) was dissolved in 20 ml of tetrahydrofuran and combined with 4.1 ml of 2 N sodium hydroxide solution. The mixture was stirred overnight at 50°C under argon. Solvent was evaporated and the residue chromatographed on 175 g of silicic acid. The column was eluted sequentially with 3 liters each of the lower phases of 2:1:1 and 1:1:1 v/v/v mixtures of chloroform:methanol:concentrated ammonium

hydroxide. The off-white powder obtained was dissolved in ethanol containing 5 ml of 2 N sodium hydroxide, treated with charcoal, filtered, and precipitated with acetic acid yielding 1.5 g of N-(4-aminobutyl)thyroxine as a white powder, mp 166°C (dec).

Analysis: Calculated for $C_{19}H_{20}I_4N_2O_3 \cdot H_2O$: C, 26.85; H, 2.61; N, 3.30.
Found: C, 26.47; H, 2.51; N, 3.29.

A suspension of 1.35 g (1.6 mmol) of N-(4-aminobutyl)thyroxine in 30 ml of methanol was treated with HCl gas for 2 minutes, then refluxed for 48 hours. Evaporation and recrystallization from methanol gave 1.35 g of the dihydrochloride salt of N-(4-aminobutyl) thyroxine methyl ester.

Analysis: Calculated for $C_{20}H_{22}I_4N_2O_3 \cdot 2HCl$: C, 26.14; H, 2.63; N, 3.05.
Found: C, 25.16; H, 2.66; N, 3.09.

A solution of 269 mg (0.5 mmol) of the label acid derivative and 51 mg (0.55 mmol) of N-hydroxysuccinimide in 8 ml of DMF was cooled to 0°C and combined with a solution of 112 mg (0.54 mmol) of dicyclohexylcarbodiimide in 2 ml of DMF. The reaction was allowed to warm to room temperature and stir for 48 hours. It was filtered and the filtrate added to a second solution prepared as follows.

To 477 mg (0.5 mmol) of the dihydrochloride methyl ester in 10 ml of DMF was added 126 mg (1.5 mmol) of solid sodium bicarbonate. After stirring for 10 minutes, the filtrate containing the N-hydroxysuccinimide ester of the label acid derivative was added. The final solution was stirred for 20 hours; an additional 42 mg

(0.5 mmol) of sodium bicarbonate was added and stirring continued for another 20 hours. The reaction was filtered, solvent was removed, and the residue chromatographed on 100 g of silicic acid. The column was eluted with a linear gradient of 1 L of ethanol to 1 L of 4:1 v/v ethanol:1 M aqueous triethylammonium bicarbonate. The crude product so obtained was rechromatographed on Sephadex LH-20, eluting with methanol. Recrystallization from methanol-ethyl acetate gave 191 mg of the methyl ester of (*VIII*) as an off-white powder, mp 125°C.

Analysis: Calculated for $C_{44}H_{51}I_4N_5O_{14}$: C, 38.25; H, 3.72; N, 5.07.
Found: C, 37.92; H, 4.01; N, 4.99.

A mixture of 230 mg (166 micromoles) of the ester and 8.53 ml of 7% potassium carbonate solution in 70% aqueous methanol was stirred for 24 hours, then diluted to a total volume of 22 ml with $H_2O$. The pH was adjusted to 6.0 with dilute acetic acid. The precipitate which formed was filtered and dried to give 181 mg of a tan powder. A 130 mg portion of this sample was dissolved in 2 ml of hot DMF, filtered, and diluted with 4.7 ml of anhydrous ethanol. On cooling, this gave 98 mg of (*VIII*) as a pale-beige powder, mp 203°C (dec, darkens from 180°C).

Analysis: Calculated for $C_{43}H_{49}I_4N_5O_{14}$: C, 37.76; H, 3.61; N, 5.12.
Found: C, 35.50; H, 3.43; N, 4.35.

* * * *

The above described synthesis of the β-galactosyl-umbelliferone-piperazinyl-thyroxine conjugate (*VIII*) can be modified to yield conjugates wherein $n$ = 2

through 9, $m$ = 2 through 9, $p$ = 1, $r$ = 2 through 9, and $\beta^1$ and $\beta^2$ are, independently, H or I as follows.

Conjugates having $n$ = 2 through 9 and $m$ = 2 through 9 can be made by replacing 4-(2-aminoethyl) piperazine-1-acetic acid in the synthesis of *VIII* by the appropriate 4-(*omega*-aminoalkyl)piperazine-1-alkanoic acid *L* [see Diagram 3]. Intermediates such as *L* can be prepared by reacting piperazine with one equivalent of $X{-}(CH_2)_m{-}COOC_1H_5$ where X is a leaving group as defined previously to give the mono-alkylated species *H*. These can be reductively alkylated with *omega*-hydroxyaldehydes (c.f., the previously cited reference by Hurd and Borch) to give compounds *J*, the alcohol group of which can in turn be transformed in one or more steps into a leaving group (species *K*). The latter intermediate can also be obtained by alkylating mono-substituted piperazines *H* with the difunctional reactants $X{-}(CH_2)_n{-}Y$ which were defined earlier. The transformation *K* to *L* can be accomplished by displacing the leaving group X with potassium phthalimide in a suitable solvent. The transformation *J* to *L* can be achieved by the method outlined in the Mitsunoku reference cited earlier. Intermediate *L* can be converted to *M* by stepwise removal of the ester function (mild treatment with acid or base) and the phthalimido function (hydrazine or strong acid).

Conjugates where $r$ is 2 through 9 can be prepared by replacing 4-aminobutyraldehyde diethylacetal in the synthesis of *VIII* with *omega*-aminoaldehyde diethylacetals *Q* [see Diagram 4]. Such acetals can be

Diagram 3

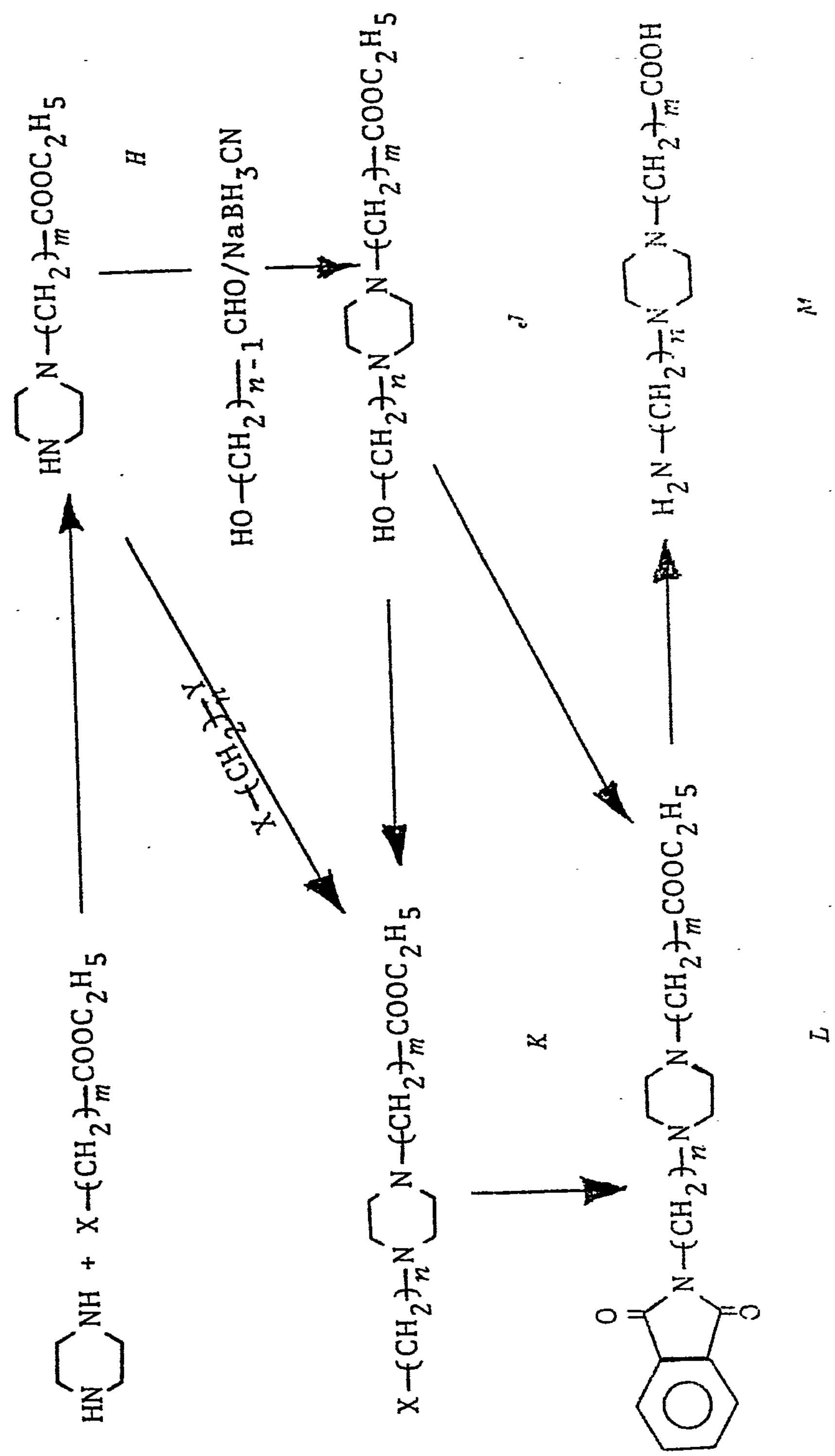


H

J

K

L

M

Diagram 4

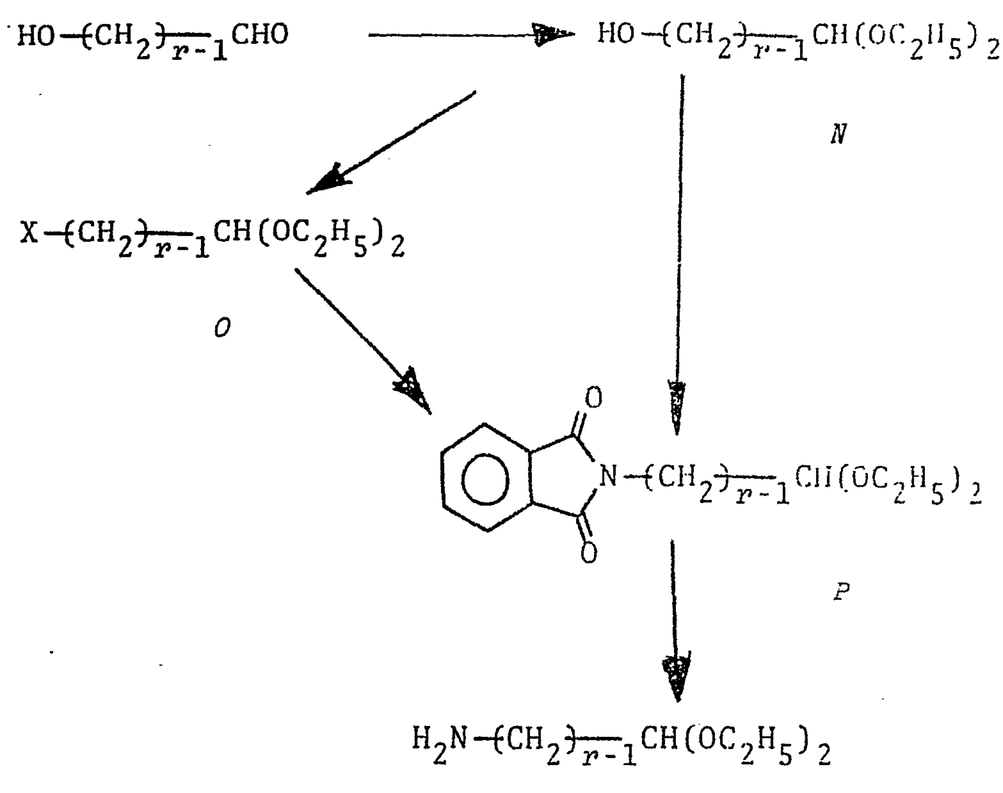

*N*

*O*

*P*

*Q*

obtained from the previously mentioned *omega*-hydroxyaldehyde acetals *N* (Hurd, *supra*). These hydroxy acetals can be converted directly to *omega*-phthalimido aldehyde acetals *P* by the previously cited Mitsunoku procedure or indirectly by transforming the hydroxy group into a leaving group (species *O*) and subsequently displacing X with potassium phthalimide. Treatment of intermediate *P* with hydrazine will produce the *omega*-aminoaldehyde acetals *Q*.

β-Galactosyl-umbelliferone-piperazinyl-thyroxine, *IX*.

The structure of this compound is shown in Fig. 2a of the drawings.

A mixture of 11.1 g (0.05 mol) of 1-(2-trifluoroacetamidoethyl)piperazine (Aldrich), 9.9 ml of 3-chloropropionaldehyde diethylacetal (Aldrich), 6.2 g of sodium bicarbonate, and 50 ml of DMSO was stirred at room temperature for 3 days. An additional 9 ml of the chloroacetal was added, and the reaction heated at 85°C for 90 minutes. The solvent was removed and the residue taken up in chloroform, washed three times with $H_2O$, dried, and evaporated. The crude product was chromatographed on 300 g of silica gel eluting with 160:10:1 v/v/v chloroform, methanol, concentrated ammonium hydroxide. This gave 3.5 g of 1-(3-diethoxypropyl)-4-(2-trifluoroacetamidoethyl) piperazine (the diethylacetal) as a yellow oil.

Analysis: Mass Spectrum (70 e.V.): m/e 355 [$M^+$]; 238 [$M^+$ minus $CH_2CH(OC_2H_5)_2$]; 299 [$M^+$ minus $CF_3CONHCH_2$].

The diethylacetal (500 mg, 1.4 mmol) was stirred at room temperature for 4 hours in 20 ml of tetrahydrofuran containing 0.4 ml of concentrated hydrochloric acid. Solvent was evaporated and the residue, now consisting of 3-[4-(2-trifluoroacetamidoethyl) piperazinyl-1]propionaldehyde (the aldehyde) was dried for 30 minutes under high vacuum. Thyroxine ethyl ester hydrochloride (1 g, 1.2 mmol) was dissolved in 15 ml of 95% aqueous ethanol. The aldehyde was added, followed by an equivalent of 20% sodium hydroxide to give a solution of pH.5.0. To this was added 300 mg (4.8 mmol) of sodium cyanoborohydride. After stirring for 3 days at room temperature the reaction product was chromatographed on 100 g of silica gel, eluting

with 160:10:1 v/v/v chloroform:methanol:concentrated ammonium hydroxide, to produce 620 mg of the $T_4$ ester intermediate as a white powder, mp 84-90°C.

Analysis: Calculated for $C_{28}H_{32}F_3I_4N_4O_5$: C, 31.45; H, 3.02; N, 5.24.
Found: C, 30.92; H, 2.80; N, 5.00.

A 600 mg (0.56 mmol) portion of the $T_4$ ester intermediate was dissolved in 1 ml of a solution made by dissolving 1.1 g of 85% potassium hydroxide in 10 ml of $H_2O$. After stirring for 48 hours at room temperature, tlc (4:4:1 v/v/v chloroform:methanol: concentrated ammonium hydroxide on silica gel) showed complete conversion to N-[[3-[4-(2-aminoethyl) piperazinyl-1]propyl]]thyroxine (the $T_4$ acid intermediate). Solvent was evaporated and the residue taken up in 10 ml of $H_2O$. The pH of the solution was adjusted to 9.0 with sodium bicarbonate and 40 ml of DMF was added.

At the same time, 230 mg (0.62 mmol) of 7-β-galactosylcoumarin-3-carboxylic acid, *supra*, and 70 mg (0.67 mmol) of N-hydroxysuccinimide were dissolved in 5 ml of DMF and cooled to 0°C. Dicyclohexylcarbodiimide (127 mg, 0.62 mmol) was added and the solution allowed to warm to room temperature and stirred for 3 hours. The precipitate of dicyclohexyl urea was removed by filtration and the filtrate added to the cold (-5°C) solution of the $T_4$ acid intermediate. The combined solution was stirred overnight. Solvent was removed and the residue chromatographed on 100 g of silica gel, eluting with 5:1 v/v ethanol: 1 M aqueous triethylammonium bicarbonate. This gave 230 mg of the thyroxine derivative (*IX*) as a white solid, mp 207-215°C (dec).

Analysis: Calculated for $C_{40}H_{44}I_4N_4O_{12} \cdot 2H_2O$: C, 36.05; H, 3.63; N, 4.20.
Found: C, 35.87; H, 3.89; N, 4.06.

* * *

The above described synthesis of the β-galactosyl-umbelliferone-piperazinyl-thyroxine conjugate (*IX*) can be modified to yield conjugates wherein $n$ = 2 through 9 and $m$ = 2 through 9 by replacing 4-(2-aminoethyl)piperazine-1-acetic acid in the synthesis of *IX* with the appropriate 4-(*omega*-aminoalkyl)piperazine-1-alkanoic acid *M* as shown for *VIII*.

B. Relative fluorescence studies

Relative fluorescence intensity was measured on an Aminco Bowman Spectrofluorometer (American Instrument Co., Silver Springs, MD) with excitation wavelength set at 400 nm and emission wavelength set at 450 nm.

The results are shown in Table B. The fluorescence data was collected for the fluorescent umbelliferone-thyroxine products of β-galactosidase cleavage of the five labeled conjugate (*V* - *IX*) shown in Figs. 2 and 2a and for a fluorescent umbelliferone derivative (designated "RAS") that was unconjugated to analyte.

The data indicates that insertion of the N,N'-piperazinylene group into the linking arm allows the fluorescer label to express about 20% of its normal fluorescence (unconjugated, i.e., fluorescence of umbelliferone derivative).

TABLE B

| Compounds | Linking arms | Relative Fluorescence |
|---|---|---|
| RAS[1] | None | 100% |
| *V* | $-(CH_2)_4-$ | 3% |
| *VI* | (single bond) | 8% |
| *VII* | $-(CH_2)_5-CO-$ | 6% |
| *VIII* | 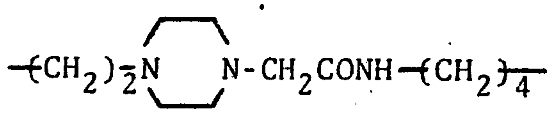 | 21% |
| *IX* | 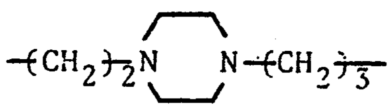 | 14% |

(1) see Table A

1. A specific binding assay method for determining an analyte in a test sample,

wherein said test sample is combined with a reagent system including a labeled conjugate comprising said analyte, or a binding analog thereof, and a photogenic label, thereby forming a reaction mixture containing a bound-species and a free-species of said labeled conjugate, said free-species yielding an analyte-photophor conjugate comprising said analyte or analog linked to a photophor capable of emitting light upon excitation,

the analyte or analog portion of said analyte-photophor conjugate capable of significantly reducing such light emission upon coming into quenching orientation with the photophor, and

wherein said photophor in said bound-species or said free-species is excited and resulting light emitted is measured as a function of the analyte in the sample,

characterized in that in said labeled conjugate said analyte or analog is joined to said photogenic label by a linking group which substantially hinders quenching orientation of said photophor with said analyte or analog portion, such linking group comprising a residue selected from piperazinylene, phenylene, cyclohexylene, bivalent-carbocyclic and heterocyclic fused-rings, acetylene, *bis*-substituted monosaccharides, bivalent bicyclooctane rod, and bivalent spiro-linked ring structures.

2. A method according to claim 1, characterized in that said linking group comprises N,N'-piperazinylene.

3. A method according to claim 1 or 2, characterized in that said photophor is a fluoroescer.

4. A method according to any of claims 1 to 3, characterized in that it is of the homogenous type wherein the photogenicity of said photophor is measurably different when said labeled conjugate is in said bound-species compared to in said free-species, and wherein said photophor is excited in said reaction mixture and the resulting emission is a function of the analyte in said sample.

5. A reagent system for the specific binding assay determination of an analyte in a test sample, comprising

(a) a binding partner for said analyte, and

(b) a labeled conjugate comprising said analyte, or a binding analog thereof, and a photogenic label,

wherein combination of said reagent system with said sample in a liquid reaction mixture results in formation of a binding partner-bound-species and a free-species of said labeled conjugate, said free-species yielding an analyte-photophor conjugate comprising said analyte or analog linked to a photophor capable of emitting light upon excitation,

the analyte or analog portion of said analyte photophor conjugate capable of significantly reducing such light emission upon coming into quenching orientation with the photophor,

characterized in that said analyte or analog is joined to said photogenic label in said labeled conjugate by a linking group which substantially hinders quenching orientation of said photophor with said analyte or analog

portion, such linking group comprising a residue selected from piperazinylene, phenylene, cyclohexylene, bivalent carboxyclic and heterocyclic fused-rings, acetylene, *bis*-substituted monosaccharides, bivalent bicyclooctane rod, and bivalent spiro-linked ring structures.

6. A reagent system according to claim 5, characterized in that said linking group comprises N,N'-piperazinylene.

7. A reagent system according to claim 5 or 6, characterized in that said photophor is a fluorescer.

8. A labeled conjugate for use in the specific assay determination of an analyte in a test sample, comprising said analyte, or a binding analog thereof, chemically joined to a photophor capable of emitting light upon excitation, such light emission being significantly reduced if the analyte or analog portion of the conjugates comes into quenching orientation with the photophor,

characterized in that said analyte or analog is joined to said photophor by a linking group which substantially hinders quenching orientation of said photophor with said analyte or analog portion, such linking group comprising a residue selected from piperazinylene, phenylene, cyclohexylene. bivalent-carbocyclic and heterocyclic fused-rings, acetylene, *bis*-substituted monosaccharides, bivalent bicyclooctane rod, and bivalent spiro-linked ring structures.

9. A labeled conjuage according to claim 8 of the formula:

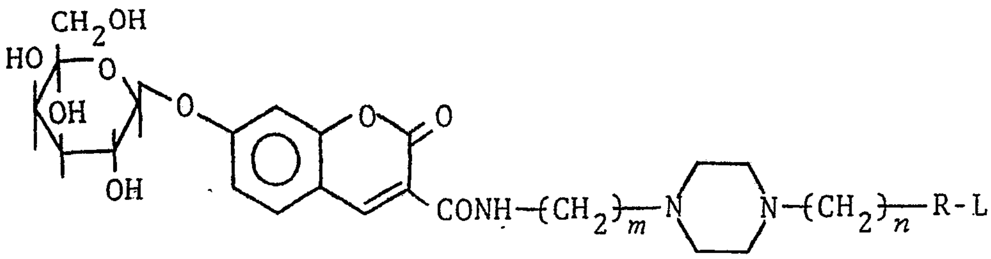

wherein *m* is an integer from 2 through 9, *n* is an integer from 2 through 9, R is a linking group, and L is an analyte or an analog thereof.

10. A conjugate according to claim 9, characterized in that L is a hapten of molecular weight less than 1500.

ALK/mc

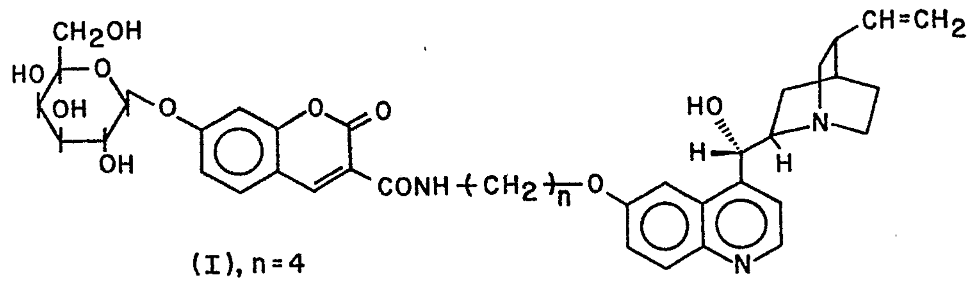

(I), n=4
(II), n=12

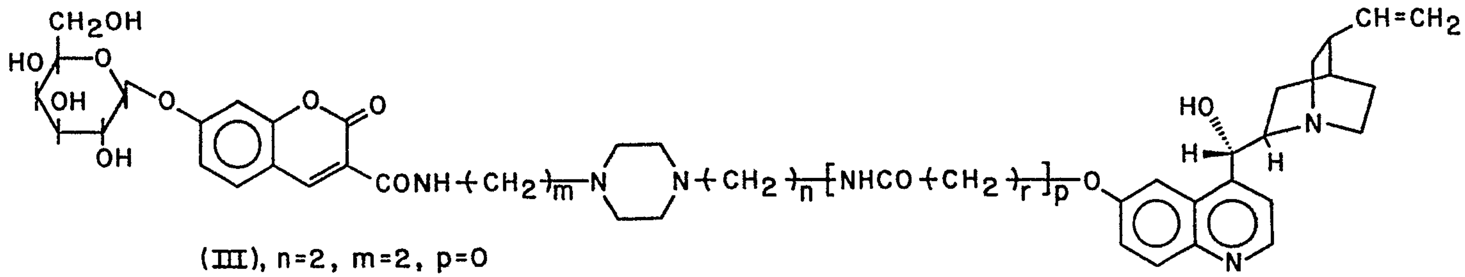

(III), n=2, m=2, p=0
(IV), n=3, m=3, p=1, r=4

FIG. I

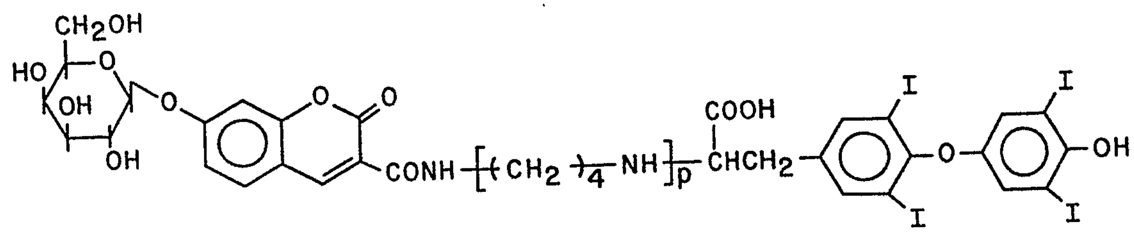

(V), p = 1
(VI), p = 0

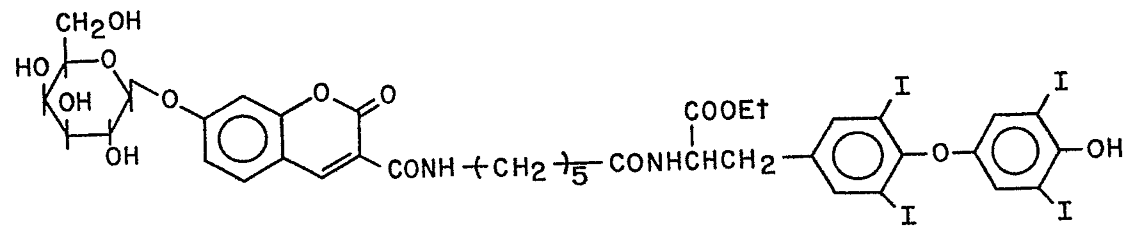

(VII)

FIG. 2

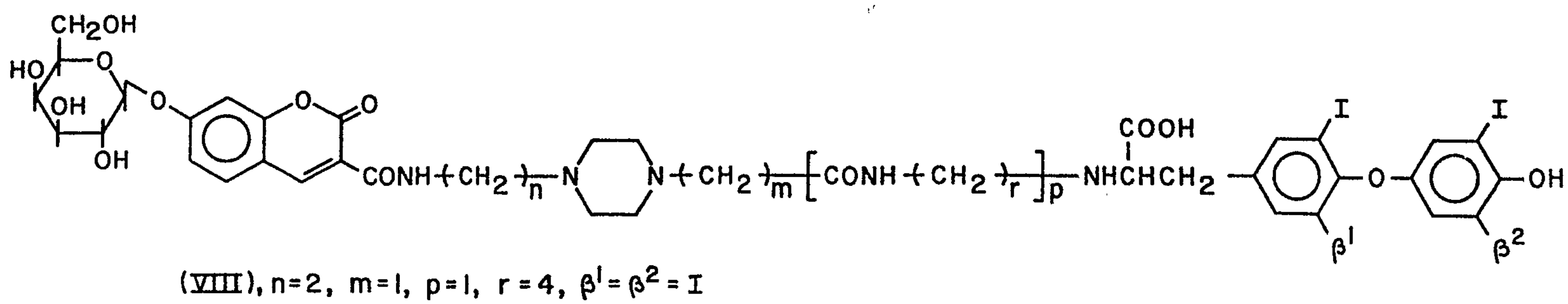

(VIII), n=2, m=1, p=1, r=4, $\beta^1 = \beta^2 = I$

(IX), n=2, m=3, p=0, $\beta^1 = \beta^2 = I$

FIG. 2a

European Patent Office

# EUROPEAN SEARCH REPORT

0087564
Application number

EP 83 10 0413

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A,D | US-A-4 279 992 (R.C. BOGUSLASKI et al.)<br>* Column 7, line 26 - column 8, line 47 *<br>--- | 1 | G 01 N 33/58 |
| A,D | US-A-4 130 462 (K.E. RUBENSTEIN et al.)<br>--- | | |
| A,D | US-A-3 996 345 (E.F. ULLMAN et al.)<br>----- | | |
| | | | TECHNICAL FIELDS SEARCHED (Int. Cl. 3) |
| | | | G 01 N 33/54<br>G 01 N 33/58 |

The present search report has been drawn up for all claims

| Place of search | Date of completion of the search | Examiner |
|---|---|---|
| BERLIN | 28-04-1983 | SCHWARTZ K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82